# EUROPEAN PATENT APPLICATION

(11) **EP 4 810 629 A1**
(43) Date of publication of application: **23.09.2026**
(21) Application number: 26180818.2
(22) Date of filing: 05.07.2022
(51) Int. Cl.: C12Q 1/6897, C12N 15/10

(54) **HIGH-THROUGHPUT CELLULAR MOLECULAR FUNCTION ASSAY SYSTEM AND METHOD**

(30) Priority: 05.07.2021 US 202163218429 P
(62) Document number(s) of the earlier application(s) in accordance with Art. 76 EPC: 22838544.9 / 4 367 236
(71) Applicant: Heligenics, Inc., Las Vegas, NV 89135 (US)
(72) Inventor: SCHILLER, Martin R., Henderson, Nevada, 89011 (US); GIACOLETTO, Christopher, Las Vegas, Nevada, 89122 (US)
(74) Representative: Brand Murray Fuller LLP

(57) **Abstract**

The invention of the present disclosure relates to methods of analyzing results of a high-throughput assay system for molecular functions and cell processes, comprising generation of a plasmid cDNA library for a gene of interest, conversion of the plasmid cDNA library into a lentiviral library, engineering of a cell line to encode a reporter assay to measure a specific molecular function or cell process, providing a population of cells, each cell comprising a gene of interest and an engineered reporter element, sorting the population of cells into a plurality of pools, performing targeted next-generation sequencing for the integrated gene cDNA and barcode to generate a plurality of paired end reads for each cell-sorted pool, and performing a bioinformatics analysis to determine the affect each mutation in the protein of interest has on its measured function.

## Description

### CROSS-REFERENCE TO RELATED APPLICATION

The present application is entitled to priority under 35 U.S.C. § 119(e) to U.S. Provisional Patent Application No. 63/218,429, filed July 5, 2021. Which is hereby incorporated by reference in its entirety herein.

### SEQUENCE LISTING

The present application contains a Sequence Listing which has been submitted in XML format via Patent Center and is hereby incorporated by reference in its entirety. Said XML file, created on July 4, 2022, is named 385404-7001WO1(00005) Sequence Listing.xml and is 4 kilobytes in size.

### BACKGROUND OF THE INVENTION

High-throughput screening (HTS) technologies have transformed biomedical sciences and many of these technologies have been effectively commercialized and impact clinical care. Most of these technologies identify cell components such as DNA, RNA, or protein species, and some assess intermolecular interactions. CRISPR/Cas9 and RNAi genome wide screens can connect a gene with a cellular or organismal process. Pathways and networks are predicted from the resulting data, but these experiments only indicate a role for a gene during a cell process, and do not conclusively assess mechanism. Previous advances in assay or screening throughput include DNA and RNA sequencing, Y2H, phage display, microarrays, and the like.

There is no high-throughput assay to broadly assess molecular functions in the context of human or mammalian cells. The molecular function are the key to understanding mechanism, disease etiology, and development of therapeutic drugs. Phage or yeast display, yeast 1- or 2-hybrid, DNA encoded libraries (DEL)s, and affinity mass spectrometry assess one general type of function, molecular interactions, although these do not assess interactions in live mammalian cells. More recently, lethality selection screens of mutant libraries in mammalian cells (called deep mutational scanning), is rapidly growing to identify loss or gain of function mutants. For example many cells with p53 mutants will not survive after two weeks of culture. The presence of variants sequenced before compared to after culturing can be used as a survival screen to identify positive mutants with some activity and infer negatives for those mutants that do not survive the screening procedure.

High-throughput assay systems have had a disproportionally large impact on uncovering how cells function, as well as how misregulation can lead to disease. As such, there is a need in the art for high-throughput assay systems that can systematically address how mutations impact molecular functions or cell processes in human cells. The current invention addresses this need.

### SUMMARY OF THE INVENTION

As described herein, the current invention relates to high-throughput methods of assaying cellular molecular function. As such, in one aspect, the invention provides a method of analyzing results of a high-throughput assay system for molecular functions and cell processes, the method comprising:
generation of a plasmid cDNA library for a gene by oligonucleotide synthesis or other mutagenesis methods; the expressed gene of interest encodes a combinatorial set of one or more amino acid substitutions; each cDNA molecule in the library also comprises a unique and random barcode;
conversion of the plasmid cDNA library into a lentiviral library or another type of library for delivery to mammalian cells;
engineering of a human or mammalian cell line to encode a reporter assay to measure a specific molecular function or cell process;
providing a population of cells, each cell comprising a gene of interest and an engineered reporter element
sorting the population of cells into a plurality of pools based on a change in a measurable property of the reporter element in response to the activity of the gene of interest;
purifying the genomic DNA from each pool and performing targeted next-generation sequencing for the integrated gene cDNA and barcode to generate a plurality of paired end reads for each cell-sorted pool;
subjecting the paired end reads to a quality control step;
fusing the paired end reads at the reverse complement overlap region to build a complete contig for the cDNA and barcode; trimming the reads and extracting the barcode sequences;
grouping the barcodes from all sorted pools and performing demultiplexing to create barcode groups of identical or closely-related barcodes;
performing variant calling on the barcode groups to one variant call format file; and
processing the variant call format file to associate amino acid substitutions encoded by each cDNA with a barcode group, determine read frequencies for each barcode, barcode group, and cDNAs encoding the same amino acid substitution(s);
   and
   calculate an activity measurement to for each barcode, barcode group, and cDNAs encoding the same amino acid substitution(s) based on its distribution among the flow-sorted pools to determine the affect each mutation in the protein of interest has on its measured function; comparing the distributions of the activities for the barcode and cell groups for each mutant to that of another set of cell groups where the cell group has wild type cDNAs without mutants, mutant cDNAs that are established loss of function mutants, or other classes of mutants; the comparison of the groups are with a statistical model to test specific hypotheses regarding the activity of each mutant.

In certain embodiments, the gene of interest is a transcription factor.

In certain embodiments, the reporter element encodes a promoter element that drives expression of a cDNA encoding a fluorescent protein.

In certain embodiments, the fluorescent protein is GFP.

In certain embodiments, the variant calling step further comprises:
performing Burrows-Wheeler indexing and alignment to a wild type reference sequence for each group of related barcodes to generate a sequence alignment and map (SAM) file;
converting the SAM file to a binary alignment and map (BAM) file;
sorting and indexing the contents of the BAM file;
aligning the contents of the BAM file to generate alignments of the variants to the wildtype; and
generating a variant call file from the alignments.

In certain embodiments, the barcode comprises 32 or more nucleotides.

In certain embodiments, the cells are mammalian cells.

In certain embodiments, the cells are an immortalized cell line.

In certain embodiments, the cell sorting is accomplished by flow cytometry.

In certain embodiments, the population of cells comprises an average of hundreds of separately randomly barcoded cDNA molecules assigned to each cell group for each mutant.

In certain embodiments, each mutant has a read depth of about 2,000X to about 90,000X sequencing coverage.

In certain embodiments, the method of the above aspects or any aspect or embodiment disclosed herein further comprises validating the method by comparing the activity of a subset of mutants analyzed by the method to previously determined results.

In certain embodiments, the method of the above aspects or any aspect or embodiment disclosed herein further comprises validating the method by comparing true negatives as determined by the method to true negatives determined by an independent method.

In certain embodiments, the method of the above aspects or any aspect or embodiment disclosed herein further comprises validating the method by comparing the activity of a subset of mutants analyzed by the method to independent testing of a set of separate clones.

In certain embodiments, the method of the above aspects or any aspect or embodiment disclosed herein further comprises validating the method by comparing method results among different samples.

In certain embodiments, the method of the above aspects or any aspect or embodiment disclosed herein further comprises comprising validating the method by comparing method results in two different cell lines.

In certain embodiments, the statistical significance of mutants analyzed in the method has a median p value of p < 9 x 10⁻²⁰.

### BRIEF DESCRIPTION OF THE DRAWINGS

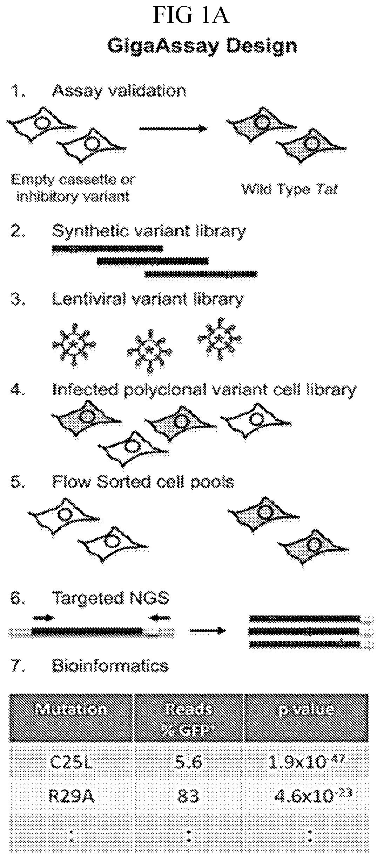

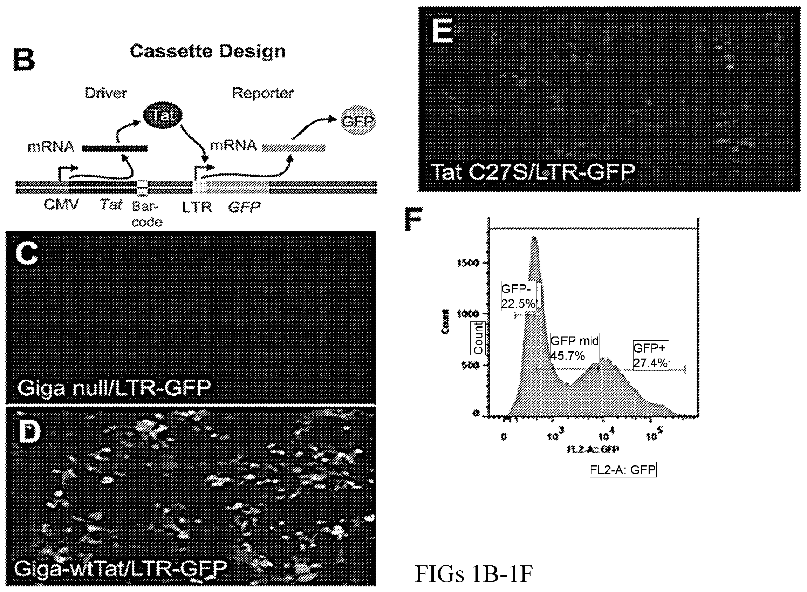

FIGs 1A-1F depict the design and implementation of the high-throughput cellular molecular function assay system for Tat transcriptional activation. FIG. 1A. Design of high-throughput cellular molecular function assay system and method system. Propagation of the recombined cells under poison selection. Cell sorting based on GFP reporter expression. gDNA is isolated, and a targeted Tat amplicon library is prepared and sequenced by NGS. FIG. 1B. Schematic representation of Tat dependent LTR transactivation inducing GFP expression. FIGs 1C-1F. Epifluorescence microscopic images of LentiX293T/LTR-GFP cells transfected with high-throughput cellular molecular function assay system and method plasmids: null/LTR-GFP (FIG. 1C. - control); wtTat/LTR-GFP (FIG.1D, + control); and an inhibitory mutant, C27S-Tat/LTR-GFP (FIG. 1E, - control)). FIG. 1F. Flow cytometry separation of high-throughput cellular molecular function assay system and method LentiX293T/LTR-GFP cell library cells with gates defined by - and + controls.

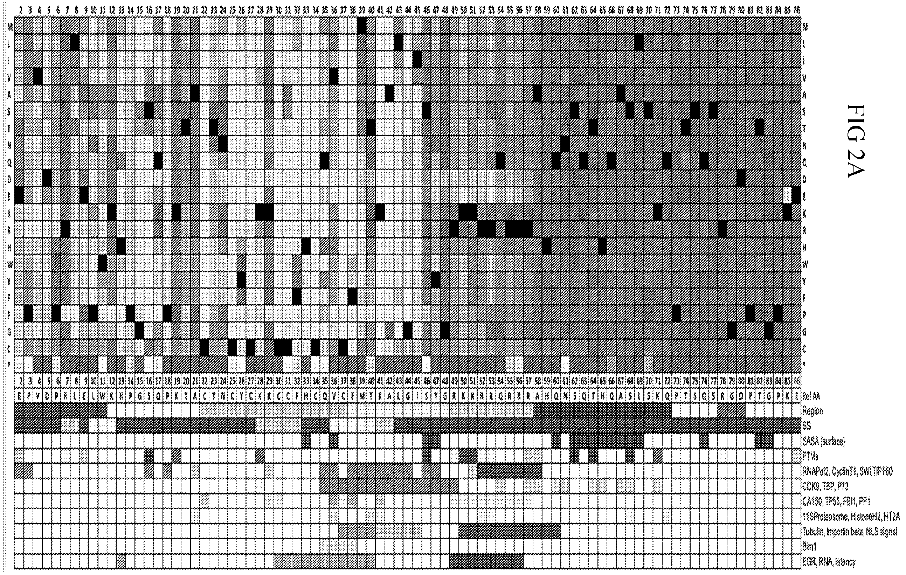

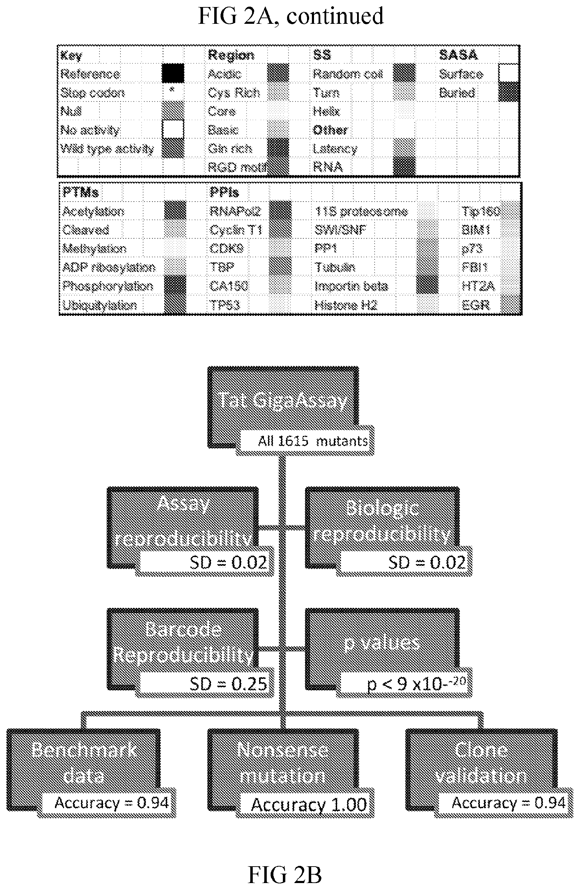

FIGs. 2A-2B illustrate a heatmap showing Tat transactivation activity for a saturating mutagenesis high-throughput cellular molecular function assay. FIG. 2A. Heatmap for mutated amino acid for each position in Tat. The shading gradient represents the level of Tat transactivation activity score measured by GFP+ / (GFP+ + GFP-) reads for each barcode averaged for each mutant. Black boxes are the wild type amino acids and dark grey boxes are null values. A high ratio (darker grey) indicates a strong enhancer and a low ratio (lighter red) indicates a strong inhibitor. A key is shown to the right. FIG. 2B. Assay reproducibility and validation.

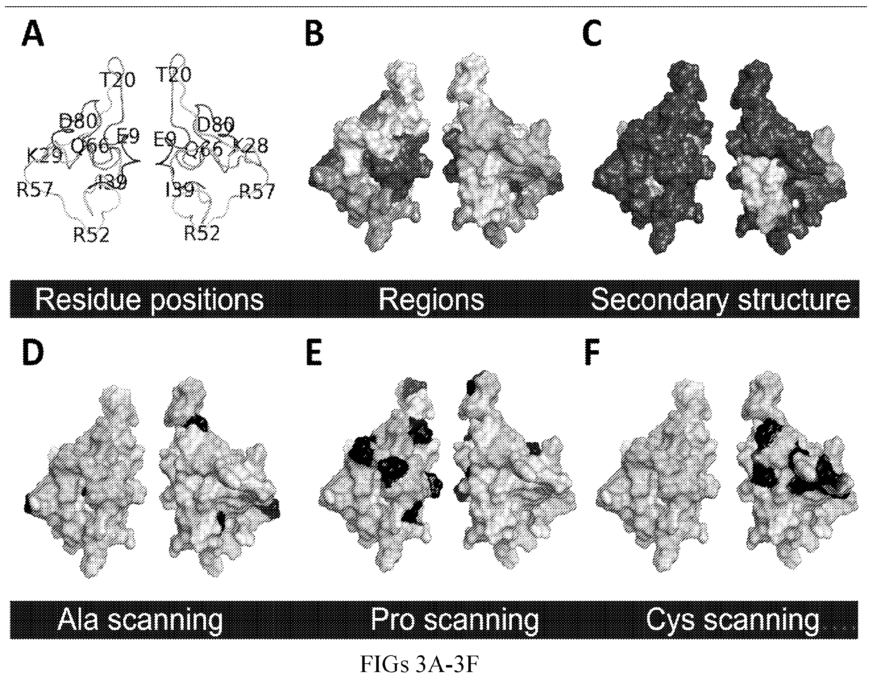

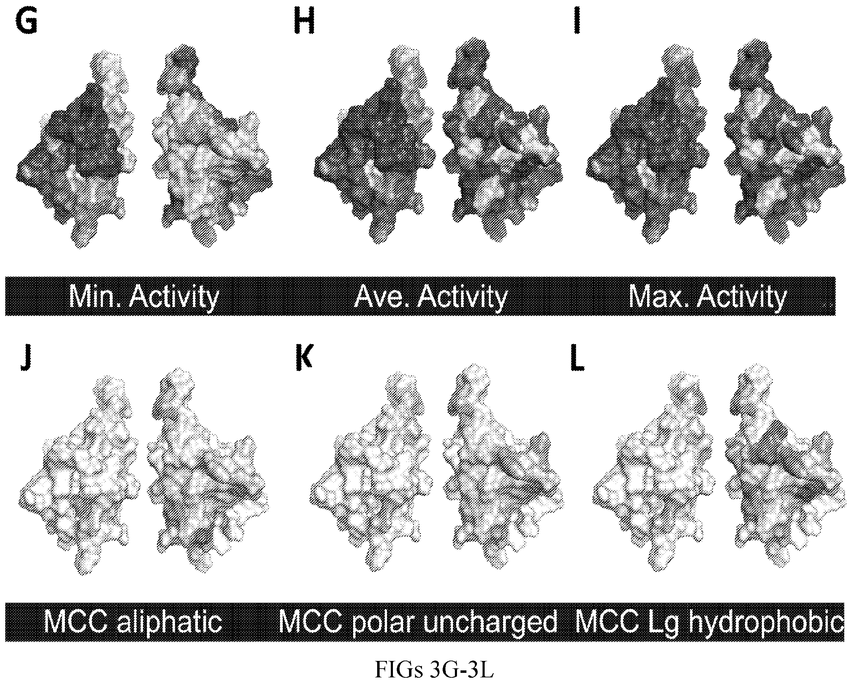

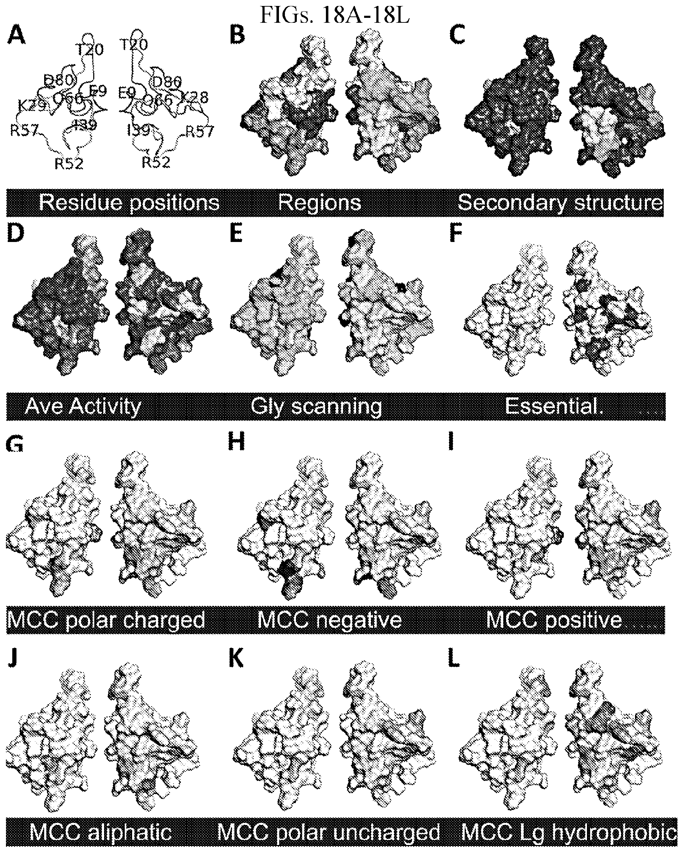

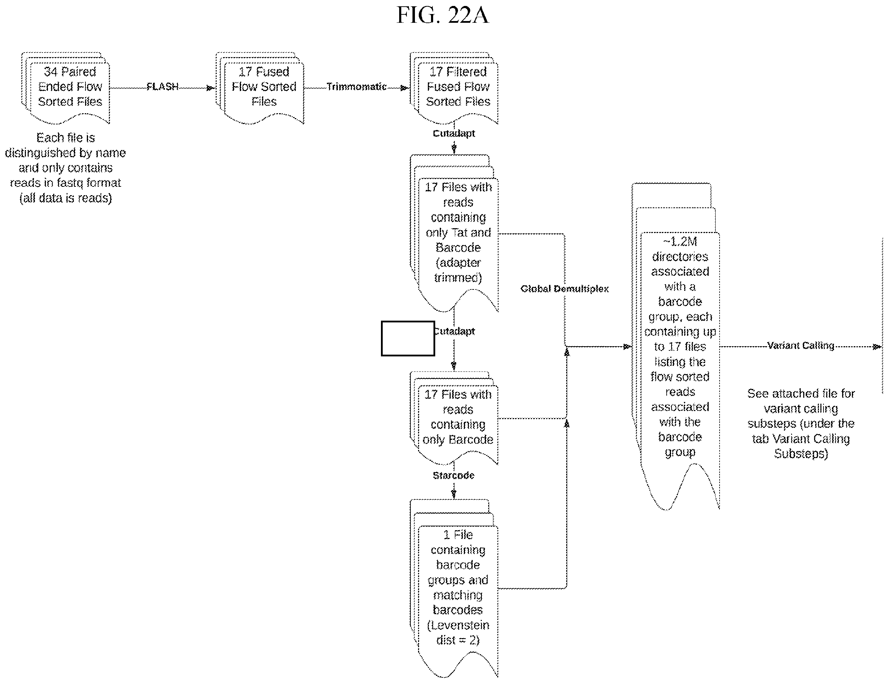

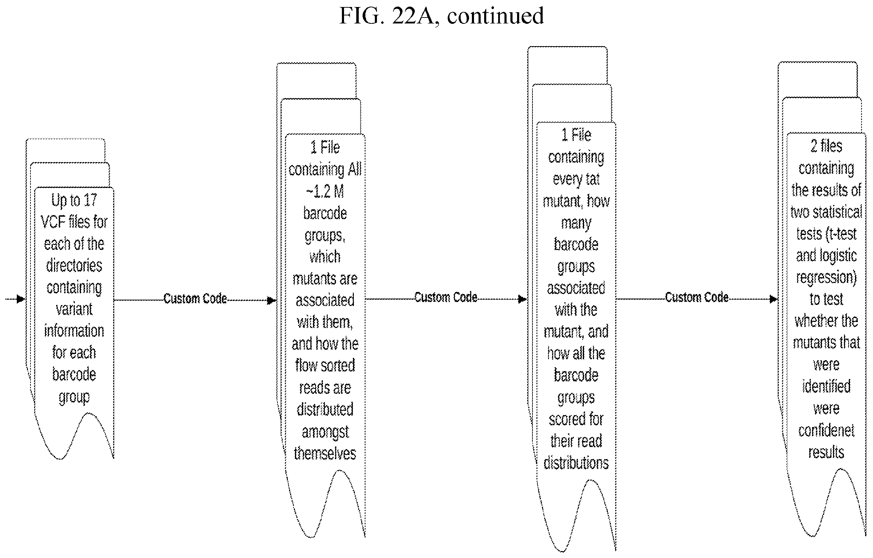

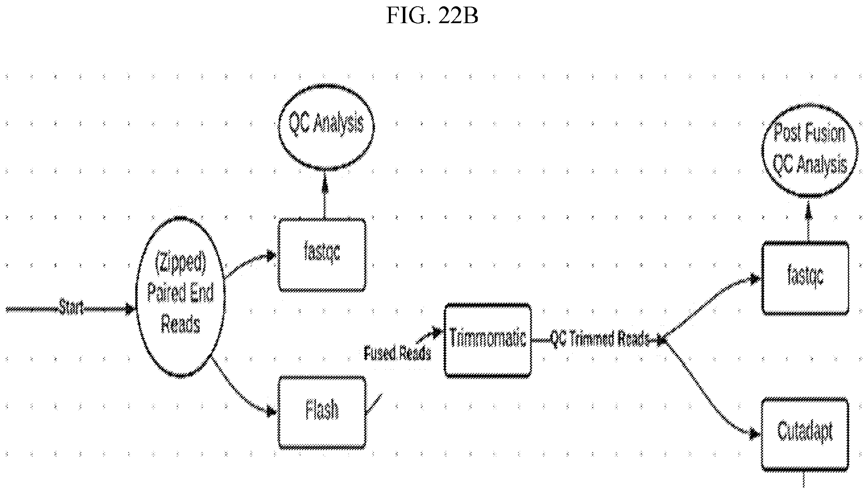

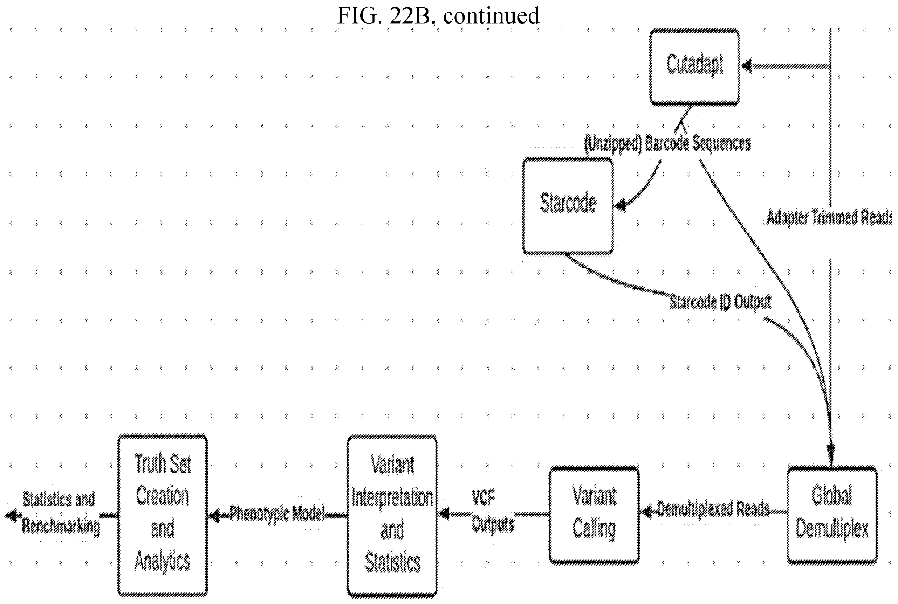

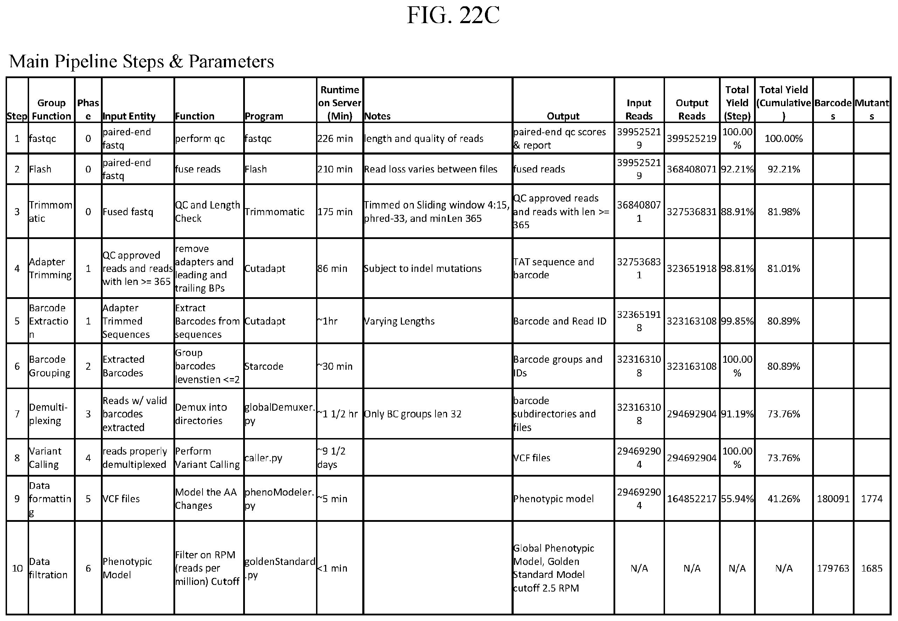

FIGs. 3A-3L illustrate tat mutant impact on structure/function. All surface maps are on the wtTat 3D structure (PDB: 1TEV) with one member of each pair rotated 180° about the Z axis: FIG. 3A. Amino acid positions on Tat backbone. FIG. 3B. Regions of Tat. FIG. 3C. Secondary structures. FIG. 3D. Ala scanning substitutions. FIG. 3E. Pro scanning substitutions. FIG. 3F. Cys scanning substitutions. FIGs. 3D-3F. Residues colored black are for reference amino acids that match the type of scanning. A gradient of yellow with no activity to green with full activity is shown. Gly scanning is shown in Fig. 18E. Minimum (FIG. 3G), average (FIG. 3H), and maximum (FIG. 3I) transactivation activity heatmap for all substitutions. A gradient of red with wild type activity to yellow with no activity is shown. Positions that do not tolerate any substitution are shown in Fig. 18F. FIG. 3J, FIG. 3K, FIG. 3L. PAS surface plots for small aliphatic, polar uncharged, and large hydrophobic amino acids, respectively (see Methods). A gradient of dark grey to white to lighter grey ranging from lower to higher accuracy for each position for the class of amino acids indicated is shown. Residues where all substitutions are inactive (C25, C27, C30, C33, C34, C37, and K41) are colored dark grey. Accuracies for polar charged amino acids, and those separated by positively and negatively charged amino acids (FIGs. 18G-18I). The color key is as in the FIG. 2A. For comparison, truncation mutants, PTMs, and PPIs are shown in FIGs. 22E-22I. Abbreviations are: Single letter amino acid code, AA = amino acid, SS = secondary structure, SASA = solvent accessible surface areas, PTM = post translational modification, NLS = nuclear localization signal, PPI = protein-protein interaction.
FIG. 4 illustrates flow cytometry optimization for Tat transactivation of LTR-GFP in Jurkat/LTR-GFP cells. A key is shown.
FIG. 5 depicts a flowchart of the GigaAssay bioinformatics pipeline. Programs are indicated in blue italic font.
FIGs. 6A-6D: Activity summary of Tat mutants. FIG. 6A, FIG. 6B. Tat mutant activity distribution in LentiX293T/LTR-GFP (FIGs. 6A, 6B) and cells on a pie graph (A) and bin plot (B). FIGs. 6A, 6B. Tat mutant activity distribution in Jurkat/LTR-GFP cells on a pie graph (FIG. 6C) and bin plot (FIG. 6D)
FIG. 7 illustrates flow cytometry chromatograms of stable LentiX293T/LTR-GFP cell lines with different Tat mutants. Mutation and percent GFP positive cells are indicated. The red peak is the Tat mutant and cyan peak is wild type cells.
FIGs. 8A-8B depict Scatter plots for replicates. FIGs. 8A, 8B. Transcriptional activity [GFP⁺/(GFP⁻ + GFP⁺)] correlation among replicate GigaAssays in LentiX293T/LTR-GFP (FIG. 8A., R²= 0.99) and Jurkat (FIG. 8B., R²= 0.99 ) cells.
FIGs. 9A-9B illustrate heatmaps of Tat mutant transcriptional activities in Jurkat /LTR-GFP cells. FIG. 9A. Organized by physiochemical properties FIG. 9B. Organized by side chain volume. The key is repeated from FIGs. 2A-2B.

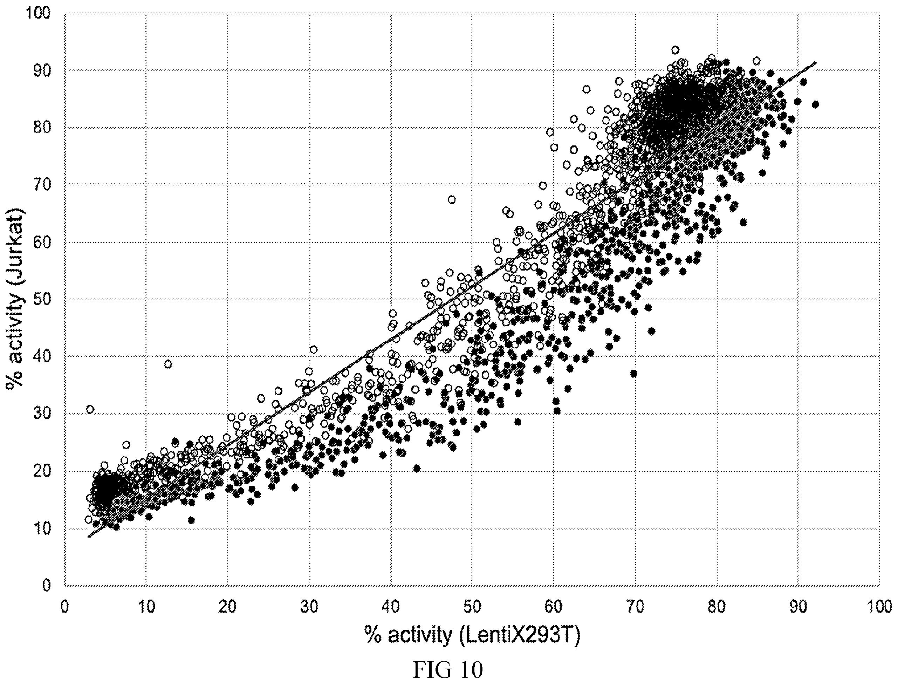

FIG 10 illustrates scatter plots comparing transcription activities for LentiX293T/LTR-GFP and Jurkat/LTR-GFP cells. Comparison of activities (percentage of reads for GFP⁺/(GFP⁻ + GFP⁺) for GigaAssays for matched mutants in LentiX293T/LTR-GFP (open circles) and Jurkat/LTR-GFP cells (blue filled circles); R²= 0.93.
FIG. 11A-11D illustrate Quantitation of GigaAssay barcodes. FIG. 11A. Heatmap of barcodes for Tat mutants in LentiX293T/LTR-GFP and Jurkat /LTR-GFP cells. A key for the heatmap colors is shown. Black cells indicate the reference sequence FIG. 11B. Barcode correlation for replicate GigaAssay samples. Each point is for a different mutant. Barcode correlation for replicate GigaAssays in LentiX293T/LTR-GFP (FIG. 11C, R²=0.95) and Jurkat/LTR-GFP (FIG. 11D, R² = 0.96) cells.
FIGs. 12A-12B depict heatmaps of reads for Tat mutants in LentiX293T/LTR-GFP (FIG. 12A) and Jurkat/LTR-GFP (FIG. 12B) cells. A key for the heatmap colors is shown. Black squares indicate the reference sequence.
FIGs. 13A-13B depict the statistical significance of activities of Tat mutants. FIG. 13A. Heatmap of p values for Tat mutants transcriptional activities in LentiX293T/LTR-GFP cells. FIG. 13B. Bin plot showing distribution of p values.
FIGs. 14A-14B depict the statistical significance of activities of Tat mutants. FIG. 14A. Heatmap of p values for Tat mutants transcriptional activities in Jurkat/LTR-GFP cells. FIG. 14B. Bin plot showing distribution of p values (n = 1615). All values are a log scale.
FIGs. 15A-15B depict heatmaps of p values Tat mutants transcriptional activities in LentiX293T/LTR-GFP cells. q values for comparison of Tat mutant activity to sets of mutants with wild type (FIG. 15A) and LOF activity (FIG. 15B). Keys for Log(p value) colors are shown.
FIGs. 16A-16B depict heatmaps of p values Tat mutants transcriptional activities in Jurkat/LTR-GFP cells. p values for comparison of Tat mutant activity to sets mutants with wild type (FIG. 16A) and LOF activity (FIG. 16B). Keys for Log(p value) colors are shown.
FIGs. 17A-17E depict Bar charts of q values for Tat mutant transcriptional activities compared to wild type Tat and LOF Tat mutants. p values for comparison of Tat mutant activity to sets of mutants with wild type activity (FIGs. 17A,17B) and reduced activity (FIGs. 17C, 17D) for LentiX293T/LTR-GFP (FIGs. 17A,17C) and Jurkat/LTR-GFP (FIGs. 17B, 17D) cells. FIG. 17E: Side by side comparison of mutant activity in LentiX293T/LTR-GFP and Jurkat/LTR-GFP. WT and LOF percentages are statistically significant (p <0.05).
FIGs. 18A-18L depict 3D maps. All surface maps are on wild type Tat 3D structure (PDB: 1TEV): FIGs. 18A-18D and FIGs. 18J-18L are repeated from FIG. 2 here for visual comparison. FIG. 18E. Gly scanning mutagenesis of Tat; black indicates wild type Gly residues). FIGs. 18D-18E. yellow color indicates wild type activity. FIG. 18F. Tat positions that do not tolerate any substitution (red) FIGs. 18G-18I. Tat PAS surface plots with each position colored with a gradient of blue to white to magenta; Coloring of residues is as described in FIG 3 and Methods. MCC = Mathews Correlation Coefficient. The color key for regions, secondary structure, PTMs, PPIs, PPVs, and Tat activity are as in FIGs. 2A-2B.
FIGs. 19A-19J depict 3D structure surface plots of different properties and function of Tat. All surface maps are on wild type Tat 3D structure (PDB: 1TEV): FIGs. 19A-19D are repeated from FIG. 2 here for visual comparison. FIG. 19E. Regions of Tat truncation and missense mutants that lose (light grey) or retain (cyan) activity. FIG. 19F. Tat PTMs. FIGs. 19G-19I. Tat PPIs in 3 groups. FIG. 19J. Solvent assessable surfaces are with residues with <10% solvent exposure colored blue. The color key for regions, secondary structure, surface, and Tat activity are as in FIGs. 2A-2B.

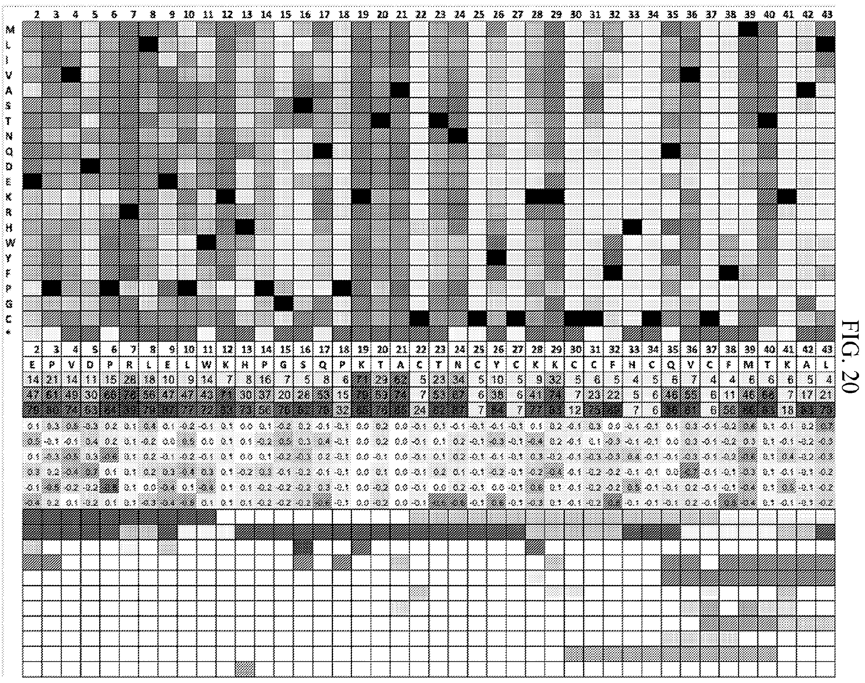

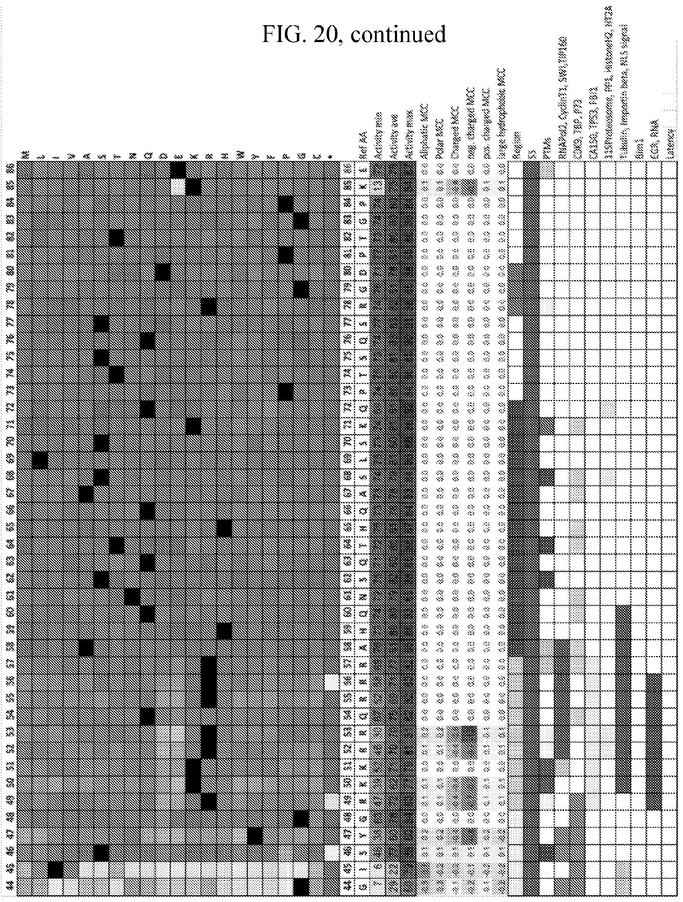

FIG. 20 illustrates a heatmap for LentiX293T/LTR-GFP cells with scores for activities and accuracies for different physiochemical groups. The color key activity heatmap are as in Fig. 2. The Mathew's correlation coefficients were used to create activity and PAS surface plots with scores ranging from -1 (blue) to 0 (white) to 1 (magenta). White indicates no specificity, Magenta indicates high specificity for the physiochemical group, and blue indicates high specificity for negative preference against the physiochemical group.
FIG. 21 illustrates a Heatmap for LentiX293T/LTR-GFP cells with amino acids ordered by side chain by volume. The color key is as in FIGs. 2A-2B.
FIGs. 22A-22C: Flow charts illustrating data processing. FIG. 22C is a table listing the main steps in the bioinformatics pipeline with input entities and output data types for each step.

### DETAILED DESCRIPTION

### Definitions

Unless defined otherwise, all technical and scientific terms used herein have the same meaning as commonly understood by one of ordinary skill in the art to which the invention pertains. Although any methods and materials similar or equivalent to those described herein can be used in the practice for testing of the present invention, the preferred materials and methods are described herein. In describing and claiming the present invention, the following terminology will be used.

It is also to be understood that the terminology used herein is for the purpose of describing particular embodiments only, and is not intended to be limiting.

The articles "a" and "an" are used herein to refer to one or to more than one (i.e., to at least one) of the grammatical object of the article. By way of example, "an element" means one element or more than one element.

"About" as used herein when referring to a measurable value such as an amount, a temporal duration, and the like, is meant to encompass variations of ±20% or ±10%, more preferably ±5%, even more preferably ±1%, and still more preferably ±0.1% from the specified value, as such variations are appropriate to perform the disclosed methods.

"Effective amount" or "therapeutically effective amount" are used interchangeably herein, and refer to an amount of a compound, formulation, material, or composition, as described herein effective to achieve a particular biological result or provides a therapeutic or prophylactic benefit. Such results may include, but are not limited to, anti-tumor activity as determined by any means suitable in the art.

"Encoding" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (*i.e.,* rRNA, tRNA and mRNA) or a defined sequence of amino acids and the biological properties resulting therefrom. Thus, a gene encodes a protein if transcription and translation of mRNA corresponding to that gene produces the protein in a cell or other biological system. Both the coding strand, the nucleotide sequence of which is identical to the mRNA sequence and is usually provided in sequence listings, and the non-coding strand, used as the template for transcription of a gene or cDNA, can be referred to as encoding the protein or other product of that gene or cDNA.

As used herein "endogenous" refers to any material from or produced inside an organism, cell, tissue or system.

As used herein, the term "exogenous" refers to any material introduced from or produced outside an organism, cell, tissue or system.

The term "expression" as used herein is defined as the transcription and/or translation of a particular nucleotide sequence driven by its promoter.

"Expression vector" refers to a vector comprising a recombinant polynucleotide comprising expression control sequences operatively linked to a nucleotide sequence to be expressed. An expression vector comprises sufficient cis-acting elements for expression; other elements for expression can be supplied by the host cell or in an in vitro expression system. Expression vectors include all those known in the art, such as cosmids, plasmids (*e.g.,* naked or contained in liposomes) and viruses (*e.g.,* Sendai viruses, lentiviruses, retroviruses, adenoviruses, and adeno-associated viruses) that incorporate the recombinant polynucleotide.

"Homologous" as used herein, refers to the subunit sequence identity between two polymeric molecules, *e.g.,* between two nucleic acid molecules, such as, two DNA molecules or two RNA molecules, or between two polypeptide molecules. When a subunit position in both of the two molecules is occupied by the same monomeric subunit; *e.g.,* if a position in each of two DNA molecules is occupied by adenine, then they are homologous at that position. The homology between two sequences is a direct function of the number of matching or homologous positions; *e.g.,* if half (*e.g.,* five positions in a polymer ten subunits in length) of the positions in two sequences are homologous, the two sequences are 50% homologous; if 90% of the positions (*e.g.,* 9 of 10), are matched or homologous, the two sequences are 90% homologous.

In the context of the present invention, the following abbreviations for the commonly occurring nucleic acid bases are used. "A" refers to adenosine, "C" refers to cytosine, "G" refers to guanosine, "T" refers to thymidine, and "U" refers to uridine.

Unless otherwise specified, a "nucleotide sequence encoding an amino acid sequence" includes all nucleotide sequences that are degenerate versions of each other and that encode the same amino acid sequence. The phrase nucleotide sequence that encodes a protein or an RNA may also include introns to the extent that the nucleotide sequence encoding the protein may in some version contain an intron(s).

The term "operably linked" refers to functional linkage between a regulatory sequence and a heterologous nucleic acid sequence resulting in expression of the latter. For example, a first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Generally, operably linked DNA sequences are contiguous and, where necessary to join two protein coding regions, in the same reading frame.

The term "polynucleotide" as used herein is defined as a chain of nucleotides. Furthermore, nucleic acids are polymers of nucleotides. Thus, nucleic acids and polynucleotides as used herein are interchangeable. One skilled in the art has the general knowledge that nucleic acids are polynucleotides, which can be hydrolyzed into the monomeric "nucleotides." The monomeric nucleotides can be hydrolyzed into nucleosides. As used herein polynucleotides include, but are not limited to, all nucleic acid sequences which are obtained by any means available in the art, including, without limitation, recombinant means, i.e., the cloning of nucleic acid sequences from a recombinant library or a cell genome, using ordinary cloning technology and PCR^{™}, and the like, and by synthetic means.

As used herein, the terms "peptide," "polypeptide," and "protein" are used interchangeably, and refer to a compound comprised of amino acid residues covalently linked by peptide bonds. A protein or peptide must contain at least two amino acids, and no limitation is placed on the maximum number of amino acids that can comprise a protein's or peptide's sequence. Polypeptides include any peptide or protein comprising two or more amino acids joined to each other by peptide bonds. As used herein, the term refers to both short chains, which also commonly are referred to in the art as peptides, oligopeptides and oligomers, for example, and to longer chains, which generally are referred to in the art as proteins, of which there are many types. "Polypeptides" include, for example, biologically active fragments, substantially homologous polypeptides, oligopeptides, homodimers, heterodimers, variants of polypeptides, modified polypeptides, derivatives, analogs, fusion proteins, among others. The polypeptides include natural peptides, recombinant peptides, synthetic peptides, or a combination thereof.

The term "promoter" as used herein is defined as a DNA sequence recognized by the synthetic machinery of the cell, or introduced synthetic machinery, required to initiate the specific transcription of a polynucleotide sequence.

As used herein, the term "promoter/regulatory sequence" means a nucleic acid sequence which is required for expression of a gene product operably linked to the promoter/regulatory sequence. In some instances, this sequence may be the core promoter sequence and in other instances, this sequence may also include an enhancer sequence and other regulatory elements which are required for expression of the gene product. The promoter/regulatory sequence may, for example, be one which expresses the gene product in a tissue specific manner.

A "constitutive" promoter is a nucleotide sequence which, when operably linked with a polynucleotide which encodes or specifies a gene product, causes the gene product to be produced in a cell under most or all physiological conditions of the cell.

An "inducible" promoter is a nucleotide sequence which, when operably linked with a polynucleotide which encodes or specifies a gene product, causes the gene product to be produced in a cell substantially only when an inducer which corresponds to the promoter is present in the cell.

The term "SAM file", as used herein, refers to a type of text file format that contains the alignment information of one or more nucleotide or protein sequences that are mapped against one or more reference sequences. These files can also contain unmapped sequences.

The term "BAM file", as used herein, refers to a file containing alignment information of various nucleotide or protein sequences that are mapped against one or more reference sequences in a binary file format. BAM files are smaller and more efficient for software to work with than SAM files, saving time and reducing costs of computation and storage.

The term "subject" is intended to include living organisms in which an immune response can be elicited (e.g., mammals). A "subject" or "patient," as used therein, may be a human or non-human mammal. Non-human mammals include, for example, livestock and pets, such as ovine, bovine, porcine, canine, feline and murine mammals. Preferably, the subject is human.

The phrase "under transcriptional control" or "operatively linked" as used herein means that the promoter is in the correct location and orientation in relation to a polynucleotide to control the initiation of transcription by RNA polymerase and expression of the polynucleotide.

A "vector" is a composition of matter which comprises an isolated nucleic acid and which can be used to deliver the isolated nucleic acid to the interior of a cell. Numerous vectors are known in the art including, but not limited to, linear polynucleotides, polynucleotides associated with ionic or amphiphilic compounds, plasmids, and viruses. Thus, the term "vector" includes an autonomously replicating plasmid or a virus. The term should also be construed to include non-plasmid and non-viral compounds which facilitate transfer of nucleic acid into cells, such as, for example, polylysine compounds, liposomes, and the like. Examples of viral vectors include, but are not limited to, Sendai viral vectors, adenoviral vectors, adeno-associated virus vectors, retroviral vectors, lentiviral vectors, and the like.

Ranges: throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

### Description

This application incorporates by reference Applicant's prior U.S. provisional patent application entitled METHODS FOR IDENTIFICATION AND CHARACTERIZATION OF SARS-CoV-2 VARIANTS, application number 63/167,702, filed 03/30/2021, and prior U.S. non-provisional patent application entitled COMPOSITIONS AND METHODS FOR STUDYING THE TAT GENE, application number 16/318,965, filed July 14, 2017, provided that if any of these prior applications or patents are in any way inconsistent with the present application (including without limitation any limiting aspects), the present application will prevail.

As described herein, the current invention relates to high-throughput methods of assaying cellular molecular function. In certain embodiments, the invention discloses the use of said high-throughput screening method by screening a HIV Tat variant library (driver), using a LTR-GFP (reporter) and thereby producing detailed functional protein map.

The high-throughput cellular molecular function assay system and method has several advantages over existing methods. The method directly assays the function of the protein. Unlike deep mutation scanning (DMS) screens, all mutations or nearly all mutations are directly measured in the high-throughput cellular molecular function assay system and method and not inferred from differences between pre-screen and screened samples. The high-throughput cellular molecular function assay of >561,000 individually barcoded mutants in living cells is at least a four order of magnitude enhancement over routine low-throughput cell based assays. For example, six Pro residues in a PxxP motifs are used to measure the impact of these motifs on Guanine nucleotide exchange factor activity. Because of the barcoding of individual molecules and high throughput at the single cell assay levels, using high-throughput cellular molecular function assay system and method allows for signal averaging large numbers of individual measurements yielding robust reproducibility, high accuracy, and a statistic for reliability of the activity for each mutant. Furthermore, all mutants are assayed under standardized conditions in the same cells with the same genetic background producing a consistency that is not seen in the literature where mutants are often studied by multiple labs with separate assay systems and conditions.

In certain embodiments of the present disclosure, the saturating mutagenesis map of Tat not only reveals shortcomings in routine interpretation of mutagenesis data, but high-throughput cellular molecular function assay system and method results create a context to improve interpretation with a new structure/function/tolerance approach. Herein, alanine scanning identifies key positions, but lacks sensitivity and misses the importance of some positions. New visualization methods of plotting saturation mutagenesis heatmap data with other structure function data, as well as physiochemical MCC surface plots enable new opportunities for visual interpretation, which will facilitate a holistic investigation of molecular functions. For example, the missense mutation analysis compared to truncation mutants shows differences that can be explained by a NLS signals. This reveals previously unrecognized limitations of truncation mutant studies. The context also produces a framework to identify ambiguities and potential misinterpretation of mutant data regarding structure, PTMs, and PPIs, especially where sites overlap with each other, as is the case for many regions of Tat.

In certain embodiments, the high-throughput cellular molecular function assay system and method of the present disclosure identifies a pattern in Tat where PPIs and PTMs are selected for robustness, while key structural elements or substitutions (e.g. proline) that impact structure are not as well-tolerated. In certain embodiments, the high-throughput cellular molecular function assay system and method of the present disclosure can be applied to other genes and assays to address other important questions in biomedical sciences.

To address this challenge, a high-throughput cellular molecular function assay system and method is disclosed. In some embodiments, the high-throughput cellular molecular function assay system and method is a single cell / one-pot assay system adapted to study how variants impact HIV Tat-driven transactivation of a green fluorescent protein (GFP) reporter. In one usage, the high-throughput cellular molecular function assay system and method is employed to assay all 1,615 Tat single amino acid substitutions with no mutant dropout. Each mutant is assayed with replicate observations in LentiX293T and Jurkat cells with an average of 100s of separately barcoded cDNA molecules and cell groups for each mutant. Each mutant had ~2,000X-90,000X sequencing coverage to measure its transcriptional activity and had a median p value of 10-20, ranging as low as 10-124. Five independent assay performance assessments with benchmark data, individually tested clones, and replicate comparisons all indicate exceptional reproducibility, accuracy, and robustness. The shortcomings of alanine scanning mutagenesis and protein truncation studies are revealed by including exhaustive substitution tolerance to the typical structure/function analysis - now structure/function/tolerance. The high-throughput cellular molecular function assay system and method is flexible and extensible and allows for a more comprehensive holistic view of protein molecular function with a highly simplified single-pot assay.

In some embodiments, a high-throughput cellular molecular function assay system and method is a mammalian cell based one-pot single cell assay where one or more variant DNA molecules are individually barcoded, assayed in cells with a fluorescent readout, flow sorted into pools, deep sequenced, and then the impact of each variant on activity is bioinformatically deconvolved. The high-throughput cellular molecular function assay system and method is ¬not a survival screen where the negatives are not directly measured. The high-throughput cellular molecular function assay system and method measures both positives and negatives for millions of individually barcoded DNA molecules producing a highly accurate and reproducible assay. The high-throughput cellular molecular function assay system and method has several other advantages over previously developed assays of cellular functions. It is flexible, readily adapted to many cell process and molecular function assays in the context of living mammalian cells. It is a high-throughput assay capable of measuring tens of thousands of reads for each of millions of individually barcoded variant DNAs, where different genotypes are pooled for each amino acid substitution, thus reliable statistical probabilities and metrics can be calculated to determine the reliability of each measurement. Because of, at least in part, the high throughput, high reproducibility among samples, and reproducibility in different cell lines, the results are highly accurate with reliable statistics. Each DNA variant molecule can be tracked through the plasmid, viral, cell libraries, and then through cell pool separation by flow cytometry.

In some embodiments, the HIV Tat transactivation of long terminal repeat-driven green fluorescent protein (GFP) expression in LentiX293T/LTR-GFP reporter cells serves as a model system for the high-throughput cellular molecular function assay system and method. LTR is the long terminal repeat in the HIV genome. This system has the advantages of an established robust reproducible assay and abundant benchmark data is published for performance assessment. Furthermore, Tat is a small gene that is suitable for assay development and of pathological significance for HIV infection and exit from latency.

### Reporter validation and library construction.

The current high-throughput cellular molecular function assay system and method approach for Tat transactivation has multiple steps. Is some instances, induction of the reporter by the Tat transgene is compared to empty vector and an inactivating mutation as controls for basal reporter expression (e.g., see FIG. 1A). Once the reporter system and cassette are validated, a barcoded plasmid library can be generated from a synthetic saturating mutagenesis dsDNA library. Each molecule in this library can be randomly barcoded and used to prepare a lentiviral variant library. A human cell line can be transduced with the lentiviral library at low MOI (0.1) to minimize double infections. A polyclonal cell library is selected for stable viral DNA integration into each cell with puromycin. Fluorescent and nonfluorescent cells are isolated into GFP+ and GFP- pools by flow cytometry. gDNA is purified from each pool, a targeted barcoded Tat amplicon can be cloned to make a Next Generation Sequencing (NGS) library for sequencing. The resulting paired-end read sequences are analyzed with a bioinformatics pipeline including one or more custom scripts to, for example, group barcodes, interpret variants, and calculate Tat transactivation activities for each mutant.

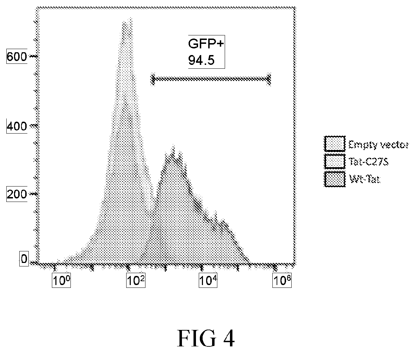

The cellular molecular function assay system and method of the present disclosure, a high-throughput cellular molecular function assay cassette encodes constitutively expressed Tat translated from a barcoded mRNA (e.g., see FIG. 1B). Tat binds to the LTR of LTR-GFP in the LentiX293T/ LTR-GFP reporter cell line where the HIV LTR drives GFP expression (e.g., see FIG. 1B). The Tat transactivation system can be tested by transient transfection of individually prepared clones transfected into separate LentiX293T/LTR-GFP cultures and visualized by epifluorescence microscopy. Cells transfected with empty vector have no detectable GFP fluorescence, while those containing wild type (wt) Tat fluoresced as expected for Tat-driven GFP expression (e.g., see FIGs. 1C, 1D). Cells transfected with a C27S mutant that inactivates Tat transactivation have minimal florescence (e.g., FIG. 1E).11 Similar results were confirmed in a Jurkat cells, a cell line derived from T cells that is a more suitable model for HIV protein studies (e.g., see FIG. 4).

To quantitate the cells and set thresholds for sorting cells expressing mutant Tat cDNAs, the method can include evaluating the same samples by flow cytometry. Cells with empty vector or the C27S mutant have low GFP expression that is not different from a control cell lacking the reporter systems, whereas cells expressing the reporter system with wtTat show strong GFP expression (e.g., see FIG. 1F). These microscopy and flow cytometry experiments validate the assay reporter system, reproducing previous observations.

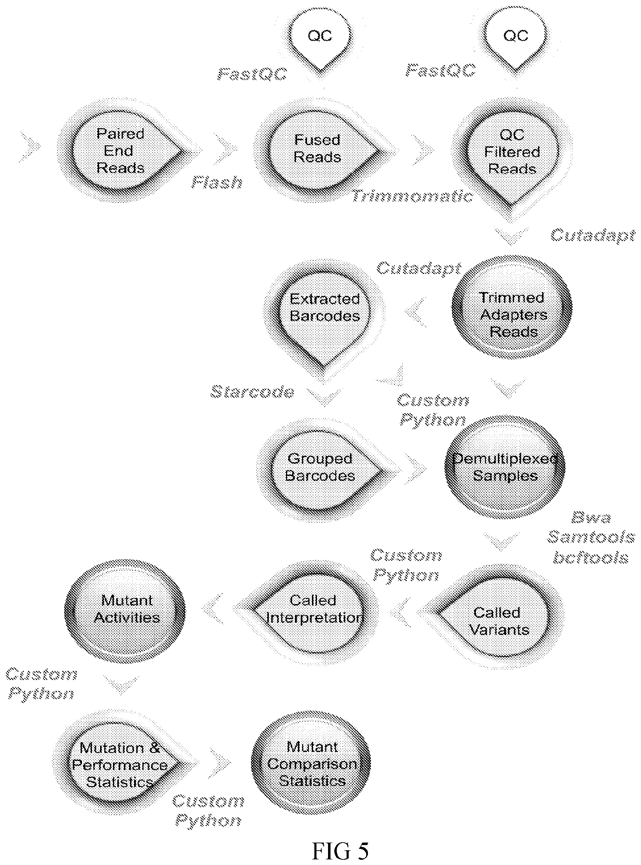

A saturating mutagenesis synthetic mutations ds-DNA Tat library (Tat accession number: AAK08486.1) is extended with synthetic 32 bp barcodes into the 3' UTR by PCR. The library is then subcloned into a lentiviral vector. NGS and bioinformatic analysis of the plasmid library shows no dropout with measurements for all 1,615 possible single amino acid substitutions. A lentiviral library is prepared by co-transfection of lentiviral vectors encoding the library of mutant Tat cDNAs into LentiX293T/LTR-GFP cells. LentiX293T/LTR-GFP cells are transduced with the lentiviral library, and after selection for stable cells, GFP- and GFP+ cells are each isolated by flow cytometry (e.g., see FIG. 1F). Pools of cells are gated based on GFP fluorescent intensities determined from negative and positive control cell samples. gDNA is isolated from each cell pool, targeted NGS libraries are constructed for the barcoded Tat cDNA, and samples are sequenced by NGS with 250 nt paired-end reads on a Illumina platform. Samples are then analyzed with a custom NGS analysis pipeline (e.g., see FIG. 5). Summary statistics for different stages of the pipeline are shown in Table 1.

### Impact of Tat single mutants on transcription

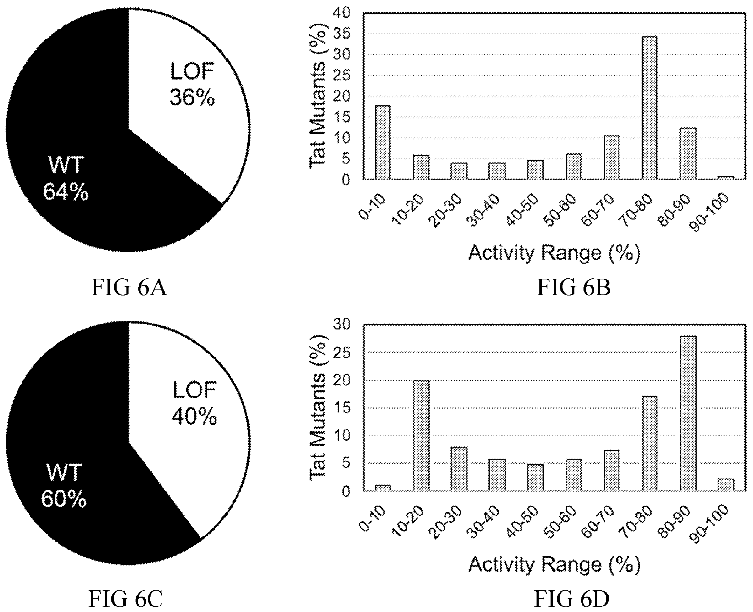

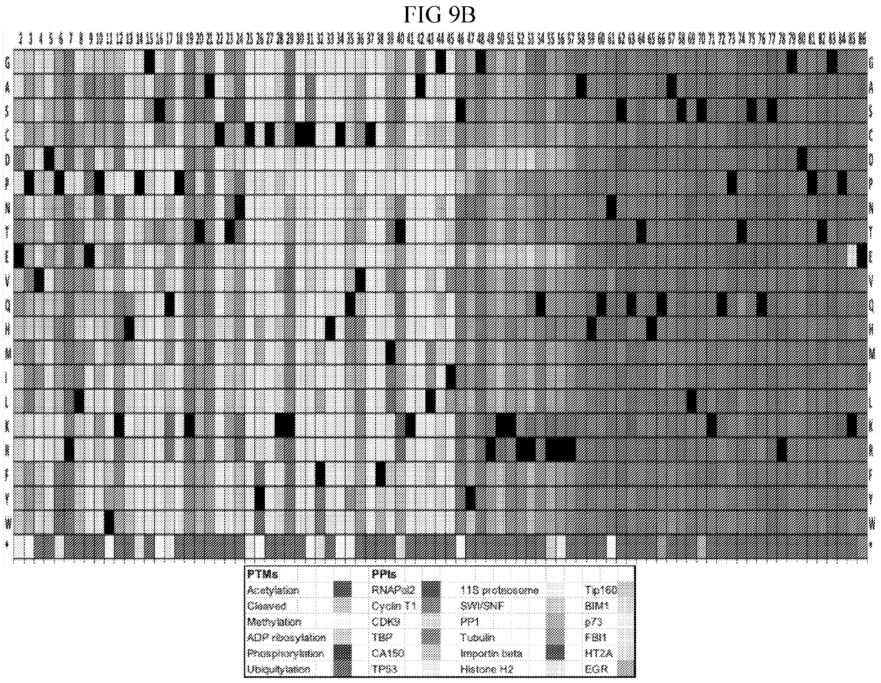

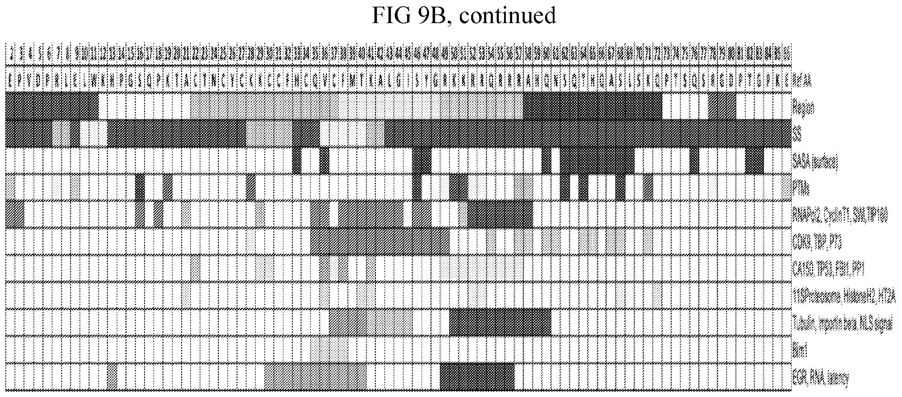

The Tat protein sequence and heatmap (e.g., see FIG. 2) show the impact of amino acid substitutions on Tat transactivation activity. The synthetic oligonucleotide and optimized cloning approach generates all possible amino acid substitutions for all positions excluding the start Met. The majority of substitutions (64%) have activities similar to wild type levels (meta p < 0.05 under Fisher's method), demonstrating a robustness for mutation tolerance in transcriptional transactivation (e.g. see FIG. 2, FIG. 6A). Approximately 36% of mutants have activities levels matching a set of known Tat mutations (meta p< 0.05 under Fisher's method), indicating a strong reduction inactivation of transcriptional activity. Of those with reduced activity, 18% have less than 10% activity of wild type indicating that these mutants are largely inactive and likely loss of function (LOF). A bin plot in FIG. 9B shows the distribution of Tat activities, relative to wtTat. Similar results were obtained among replicate samples and in Jurkat cells (e.g., see FIGs. 9C, 9D).

### High-throughput cellular molecular function assay system and method performance validation

Five independent validation tests demonstrate that the high-throughput cellular molecular function assay system and method has very high reproducibility and accuracy (e.g., see FIG. 2B). The first validation approach compares high-throughput cellular molecular function assay system and method results to benchmark data from previous reports for Tat mutant transcriptional transactivation. 442 mutants for Tat are annotated from 43 papers. Mutants are removed that have ambiguous activity reports or are no single missense mutations yielding a final list of 107 mutants from 28 papers. The high-throughput cellular molecular function assay system and method results were compared to these benchmark mutants using a threshold of 50%, with those > 50% classified as wild type activity and those <50% classified as reduced activity. Optimization is performed to a read threshold of 2.5 reads per million (rpm) based on standard performance metrics. Considering these mutant activities, high-throughput cellular molecular function assay system and method performance statistics estimates can set at: accuracy = 0.93; sensitivity = 0.94; specificity = 0.89; PPV = 0.95; and NPV = 0.89 when compared to independently published benchmark data (e.g., see Table 2).

A second validation can be based upon an independent source of true negatives measured in the high-throughput cellular molecular function assay system and method. For true negative, LOF variants, Tat nonsense variants encoding stop codons (n = 61) where each variant had more than 2.5 rpm can be examined. Tat exon 1, encoding its first 58 amino acids is, in some instances, the minimal element required for Tat transactivation activity. Nonsense variants with stop codons located less than 58 amino acids from the start Met, that have reduced activity are considered true negatives, while those 58 amino acids or longer are considered true positives with wild type activity Considering these mutant activities, the results can produce a high-throughput cellular molecular function assay system and method performance statistics for: accuracy, 1.0; sensitivity 1.0; specificity 1.0; PPV, 1.0; and NPV of 1.0 (e.g., see Fig. 2C). This analysis of intrinsic assay data can further support a high accuracy validation of the high-throughput cellular molecular function assay system and method.

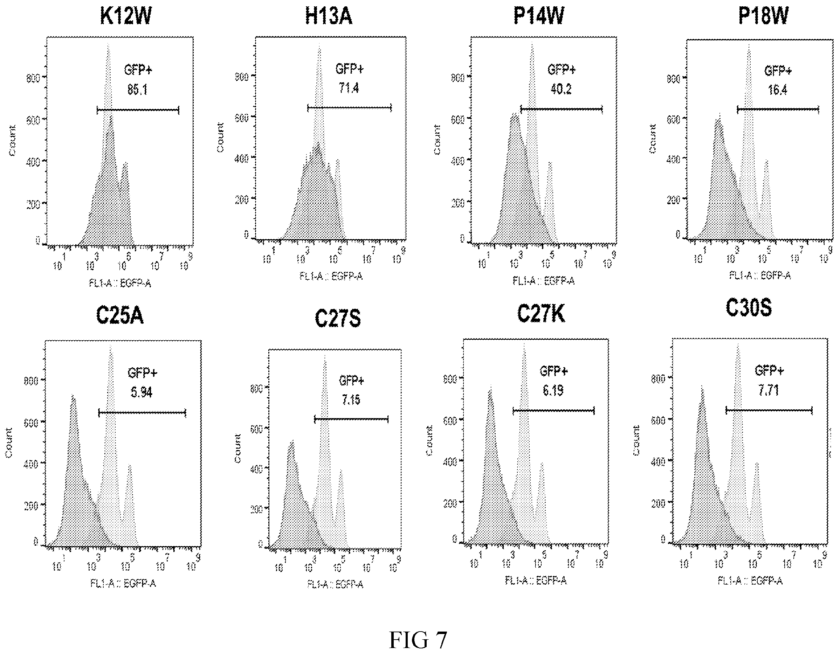

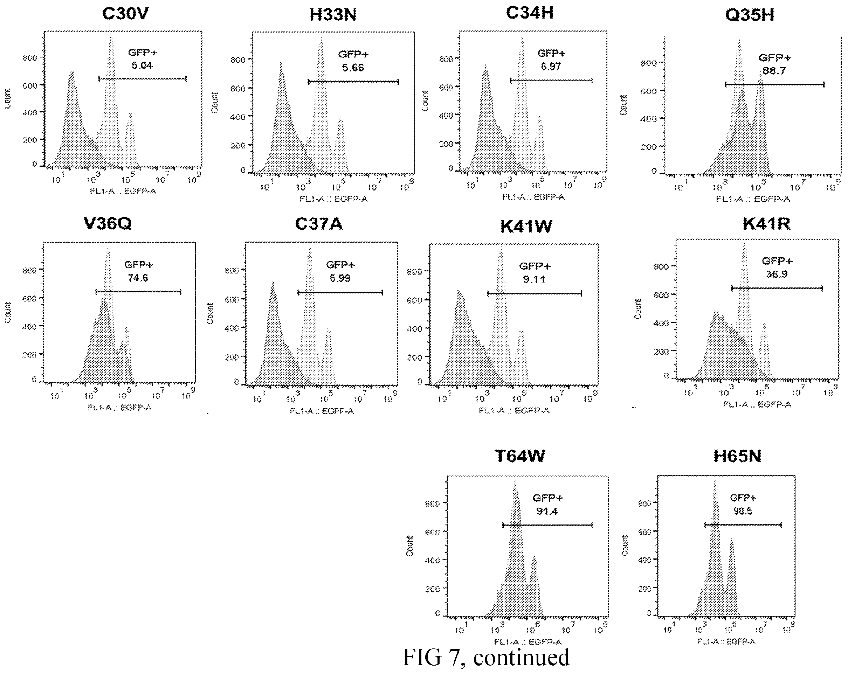

A third validation can include independent testing of a set of separate clones. Prior to the experiment 18 Tat mutants can be randomly selected, made stable LentiX293T/LTR-GFP cell lines expressing these mutants, and measured transcription activation of LTR-GFP reporter by flow cytometry. These results can be blinded until the high-throughput cellular molecular function assay system and method is complete and then compared to the high-throughput cellular molecular function assay system and method results (e.g., see FIG. 7). In some instances, the performance statistics can be: accuracy = 0.94; sensitivity = 0.75; specificity = 1.00; PPV = 1.00; and NPV = 0.92. (e.g., see Table 2).

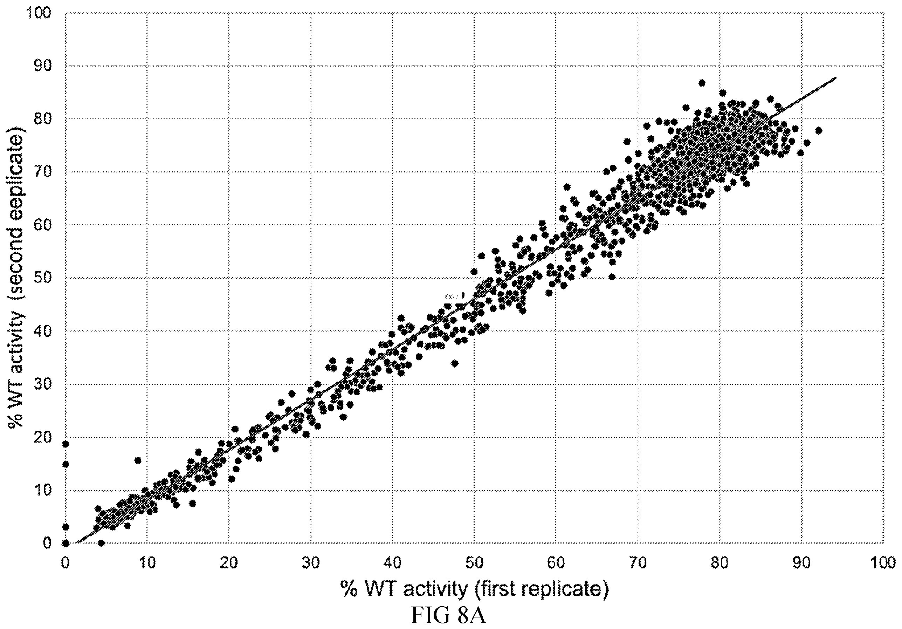

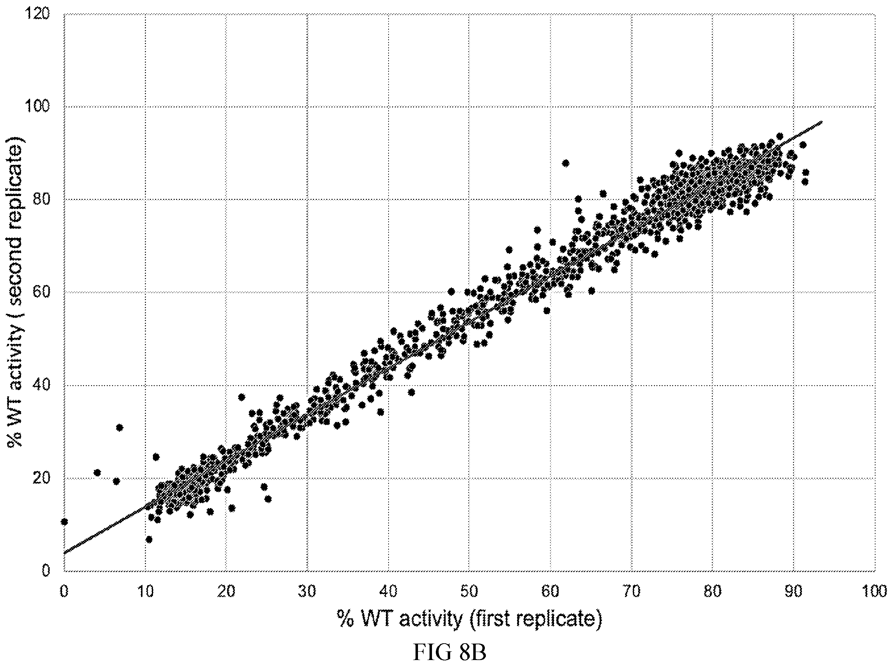

A forth validation approach can assess the reproducibility of high-throughput cellular molecular function assay system and method results among different samples. The LentiX293/LTR-GFP are transduced, selected, flow sorted, sequenced, and analyzed separately in duplicate. The global standard deviation for Tat mutant activities between technical duplicates can be very low (SD = 0.02) with 1.0 being wild type activity. Mutant activities for sample replicates for each the LentiX293 can have a very high correlation (R2 = 0.99) indicating high reproducibility (e.g., see FIG. 8A).

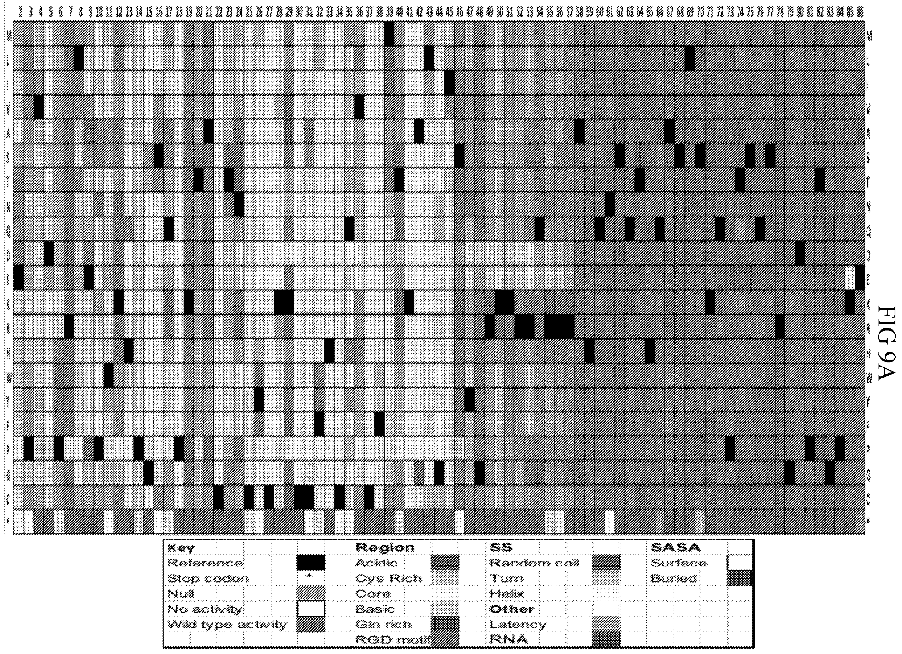

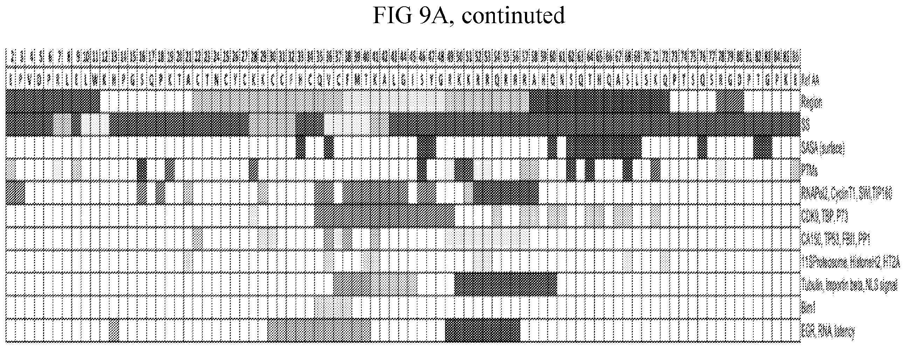

A fifth validation can compare high-throughput cellular molecular function assay biological replicates in two different cell lines (LentiX293/LTR-GFP and Jurkat/LTR-GFP cells). The high-throughput cellular molecular function assay experiment can be repeated in Jurkat cells/LTR-GFP, again with duplicate samples. Similar results for the performance statistics, reproducibility, and mutant activities may be observed (e.g., see FIG. 2C, FIGs. 8B, 9, Table 2) and an activity heatmap for Jurkat cells is shown in FIG. 9).

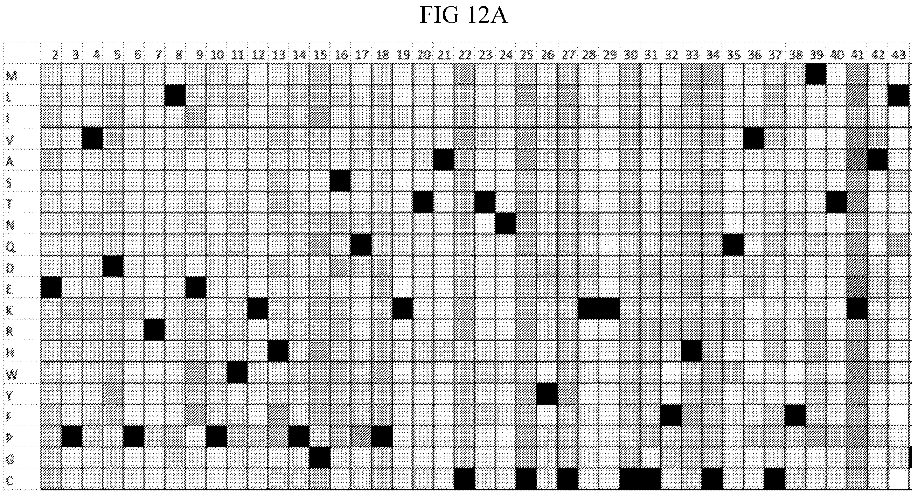

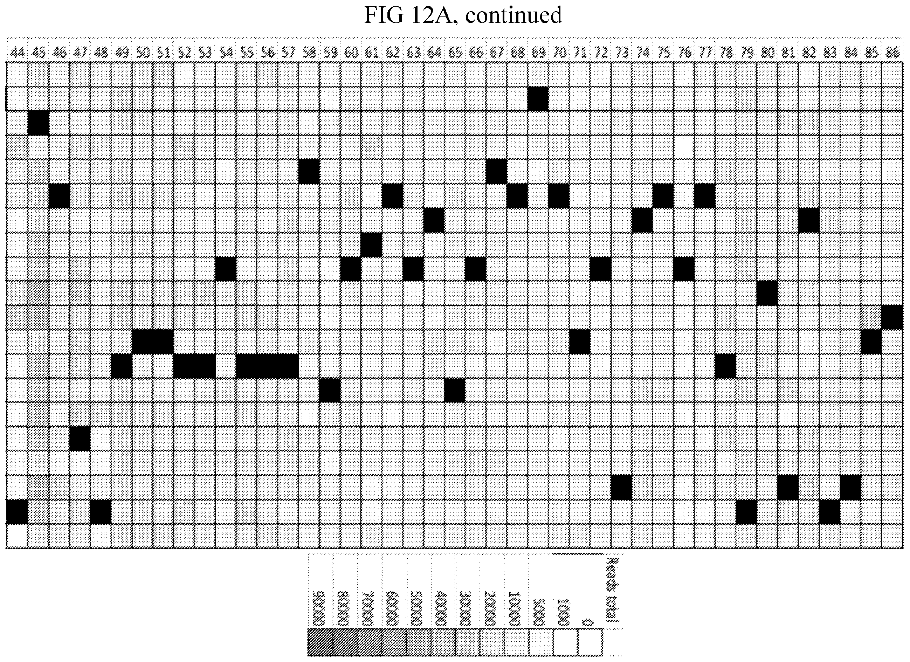

Only minor differences transcriptional activities for each mutant among the LentiX293/LTR-GFP and Jurkat/LTR-GFP cell lines indicated by comparison of activity heatmaps (e.g., see FIG. 2A and FIG. 9A) and high correlation of results for the two cells lines for matched mutants shown in a scatter plot in FIGs. 12A-12B; R2 = 0.93). Major differences can include Tat mutants with intermediate activities. Collectively, the correlation and variance analyses demonstrate reliable high data reproducibility and mutant behavior is nearly identical among replicates and between different cell lines.

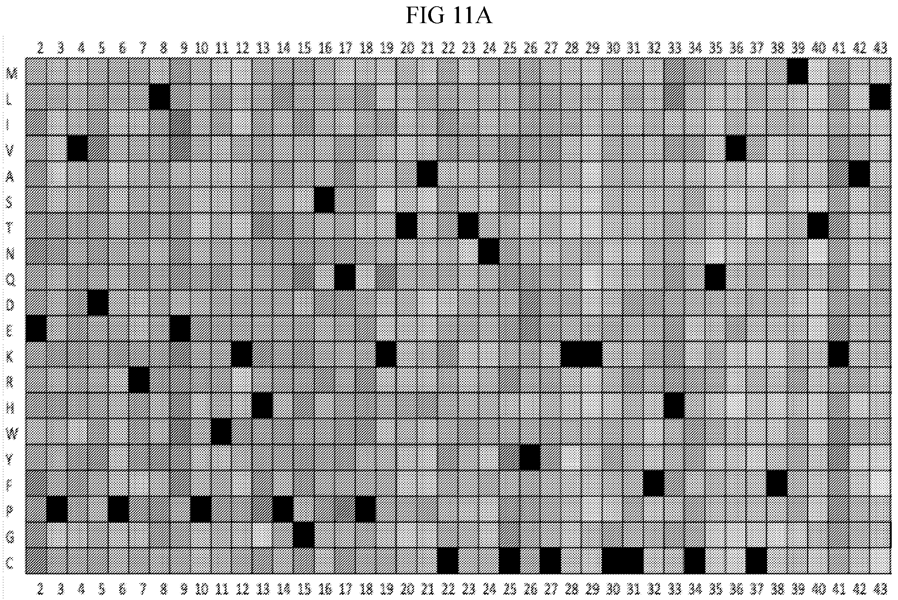

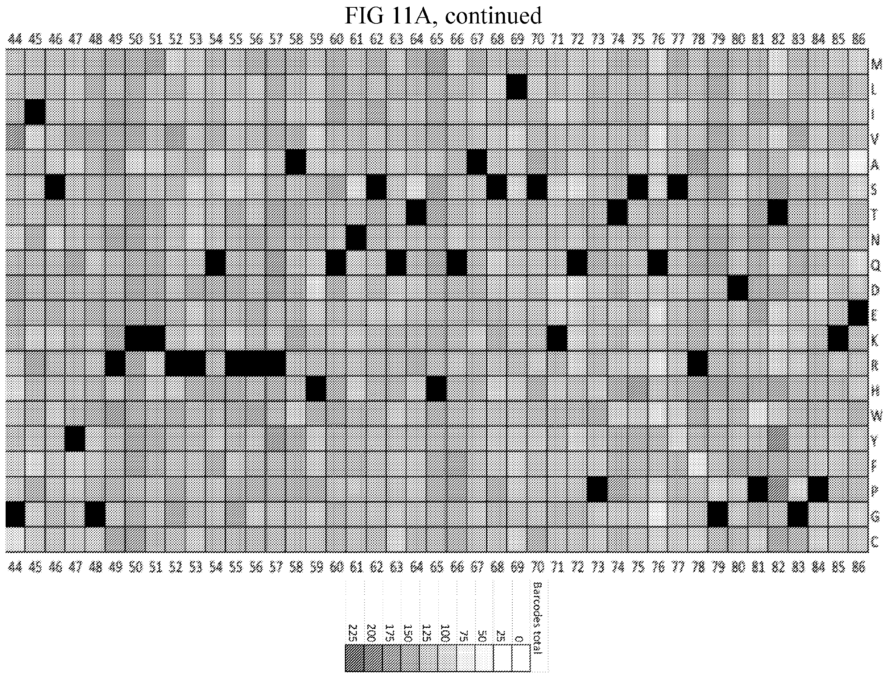

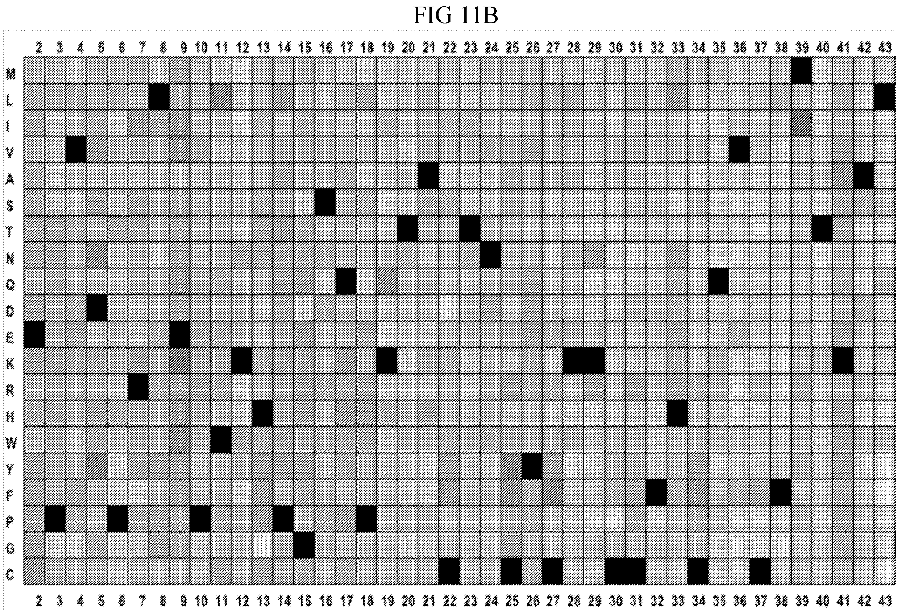

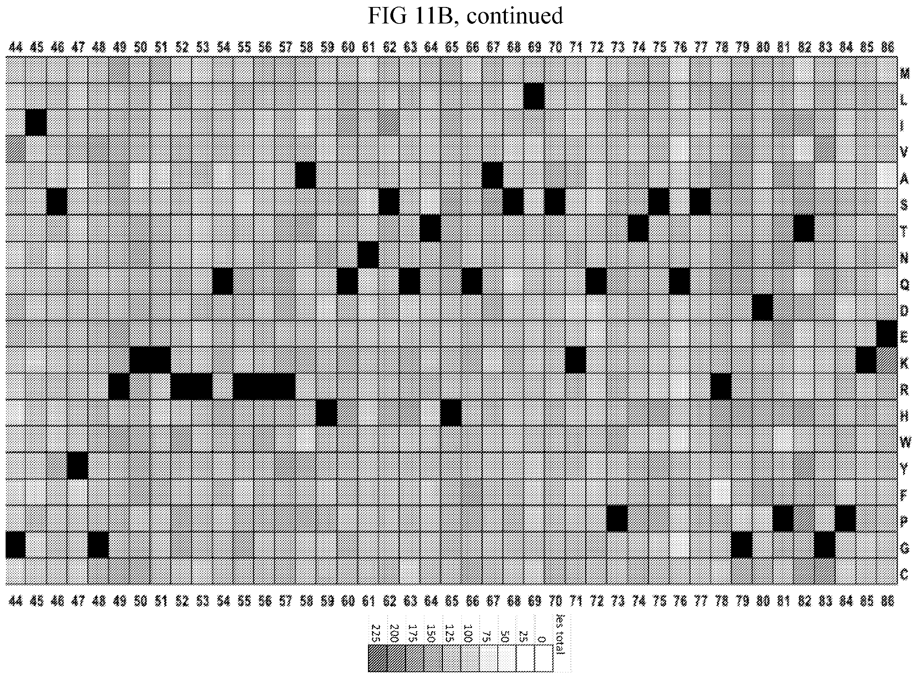

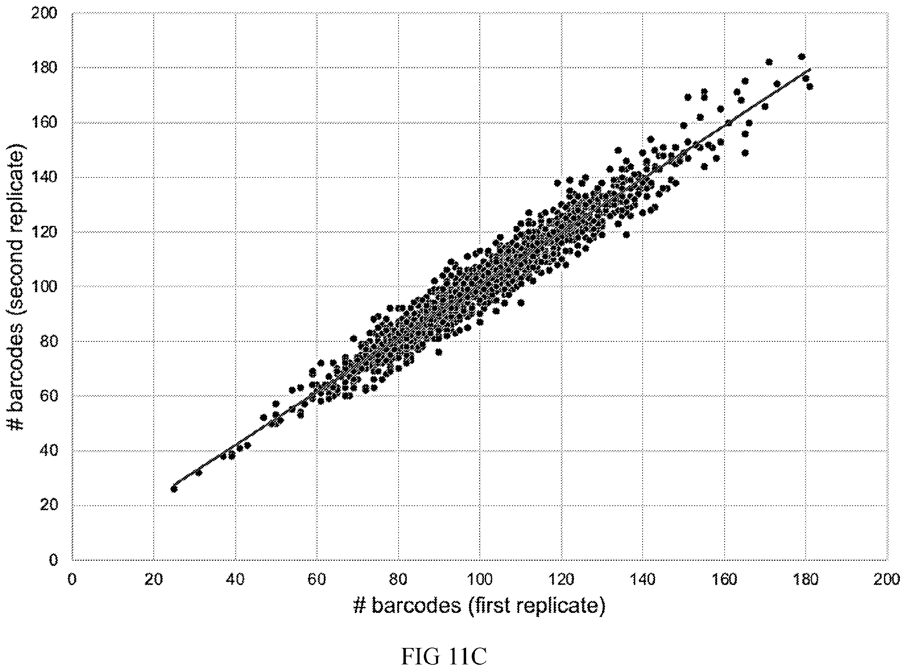

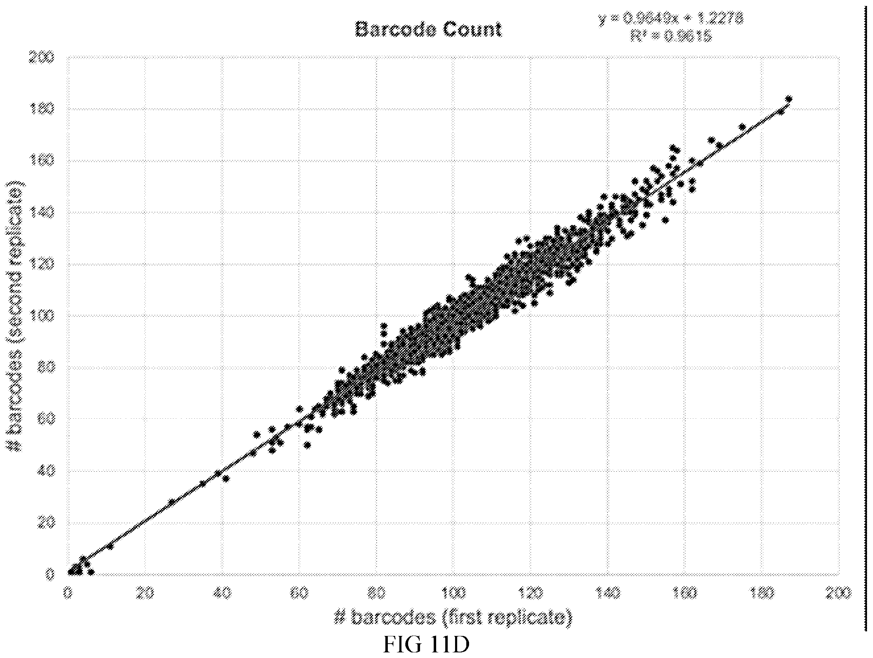

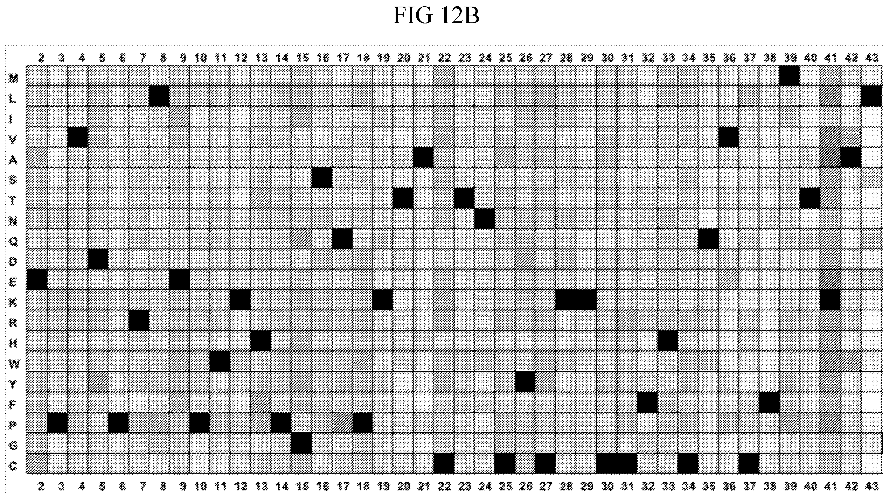

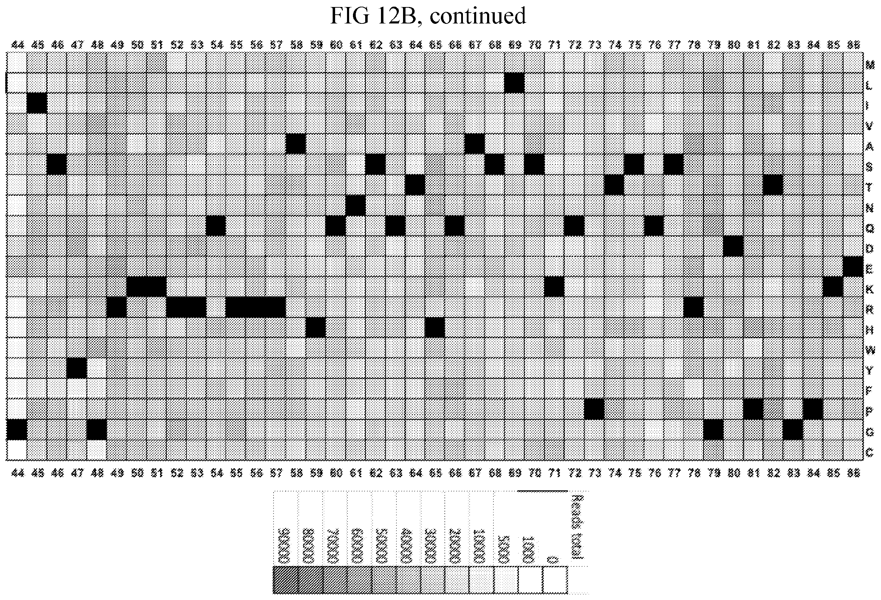

The high reproducibility is achieved from the experimental design where each individual variant cDNA has a separate random barcode which is tracked through the experiment. In some instances, during the high-throughput cellular molecular function assay system and method, each cDNA is individual barcoded and after transduction of recombinant viruses, each cell is barcoded. During selection, these cells divide forming clonal barcoded cell groups. In some instances, for the different samples and cell lines there can be ~561,000 barcoded cell groups after condensing for sequencing errors in barcodes. Each mutant in each replicate sample can have an average of 94 independent barcodes with scatter plots showing a high correlation for each replicate sample in each cell line and a heatmap for the number of barcodes for each mutant shown in (e.g., see FIG. 11). The percentage of GFP+ reads for each group is calculated from the GFP-and GFP+ reads. Each barcoded cell group has an average of 273 reads and FIG. 12 shows a heatmap with distribution of the number of reads averaging 25,662 reads per mutant for each sample that are used for variant calling and to calculate Tat transactivation activity.

The transcriptional activities for each barcoded cell group with the same mutation are averaged and used to calculate statistics. The global standard deviation for the barcoded cell groups is 0.25. While this is considerably larger than the replicate sample standard deviations, there may be multiple factors contributing to this variance such as, for example, random chromosomal lentiviral insertion sites that impact expression, cells in different phases of the cell cycle, and other types of errors when such larger datasets are analyzed at the level of individual molecules.

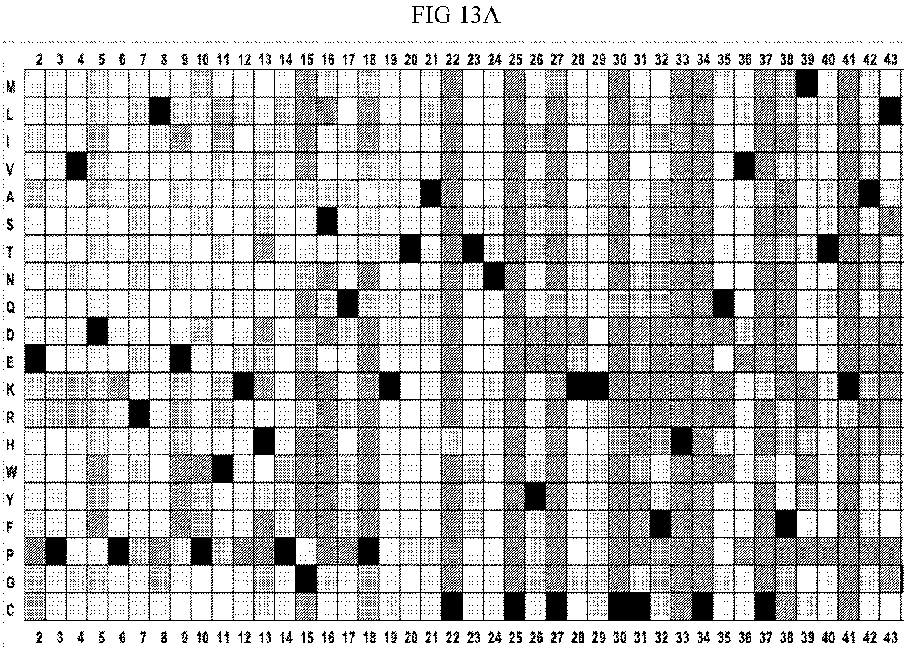

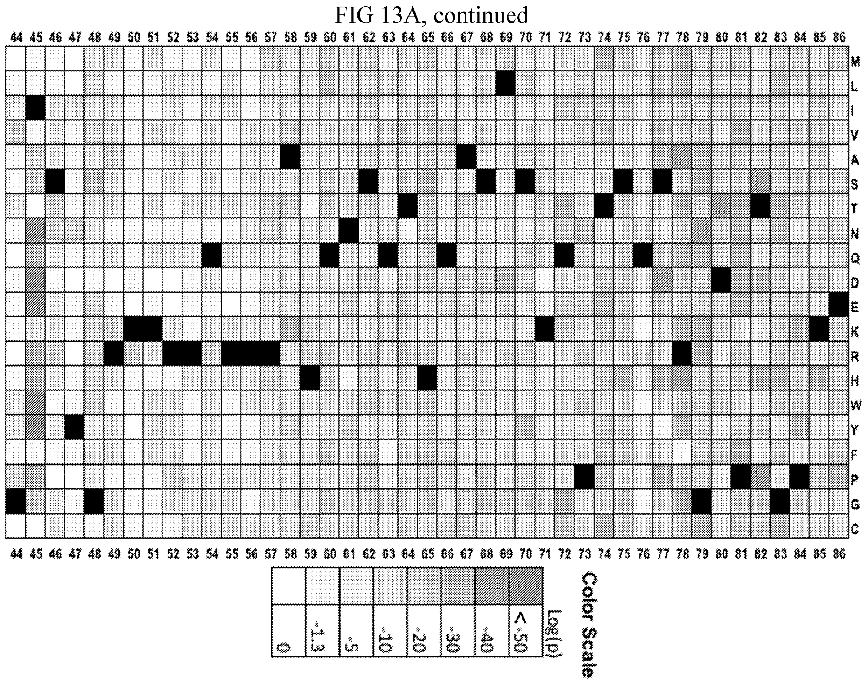

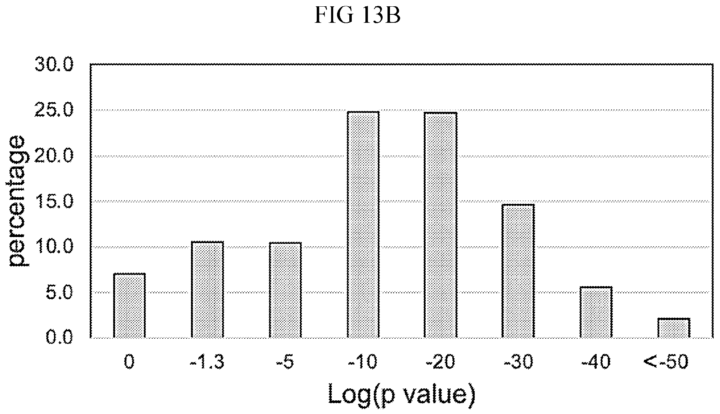

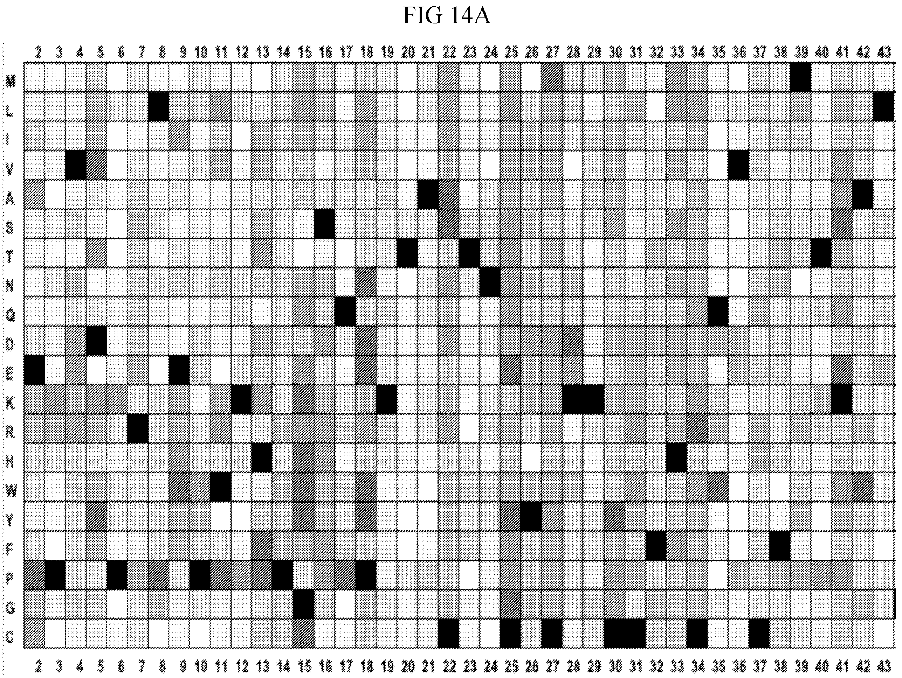

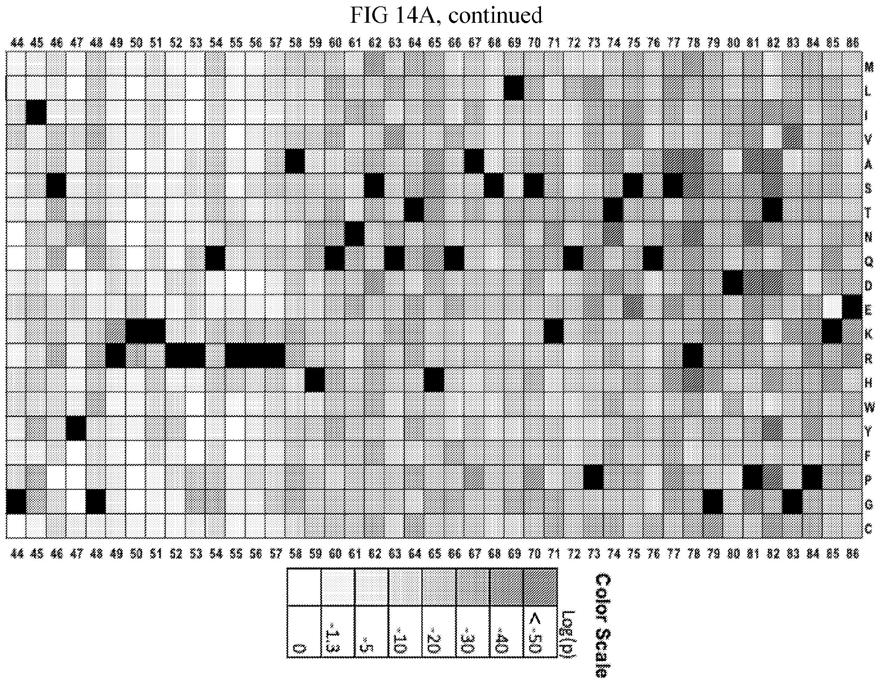

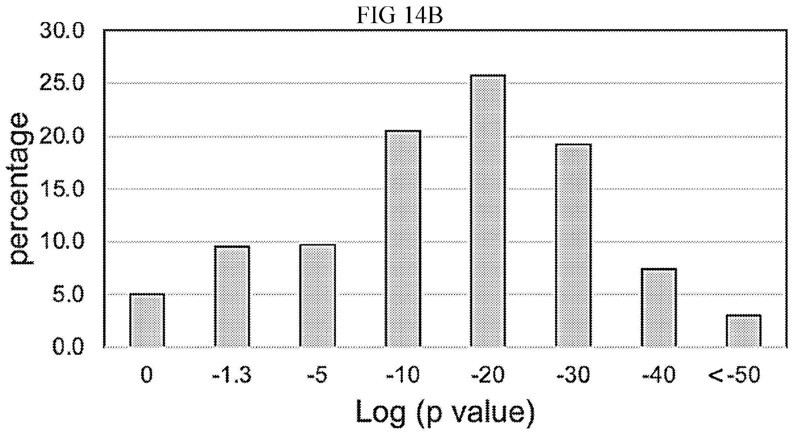

Given the breadth of data produced in this high-throughput cellular molecular function assay system and method experiment, one can reliably report metrics of confidence for the activity of each mutant. The p value for each mutant in each cell with the distributions p values frequencies are shown in Figs. 13 and 14. These heatmaps and bin plots show that most p values (95%) for mutants in both cell lines reach statistical significance. The median p value (p < 9 x 10-20) indicates significance and is quite low due to the large number of barcode replicates with an average (n=184). Some mutant p values range as low as 10-124.

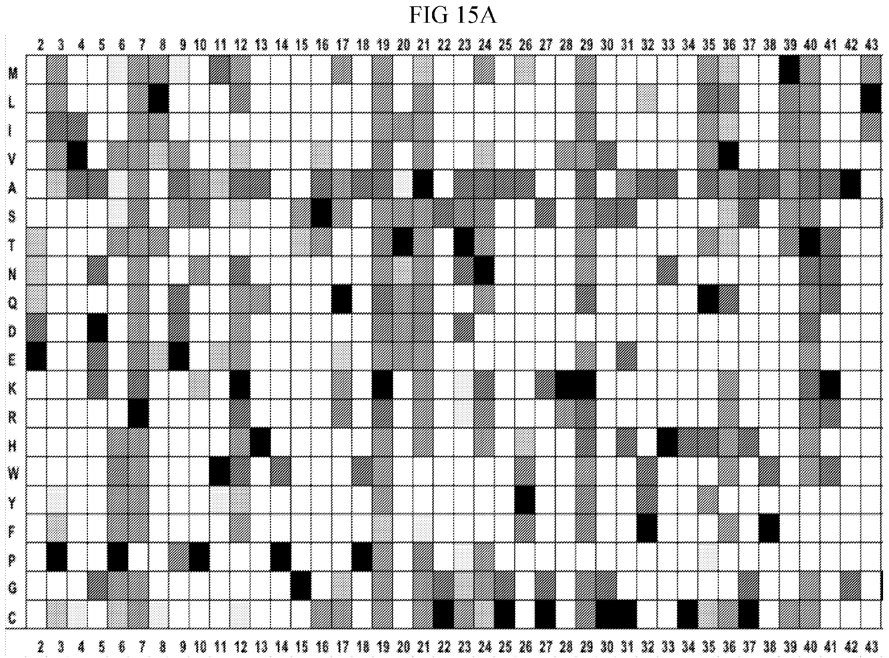

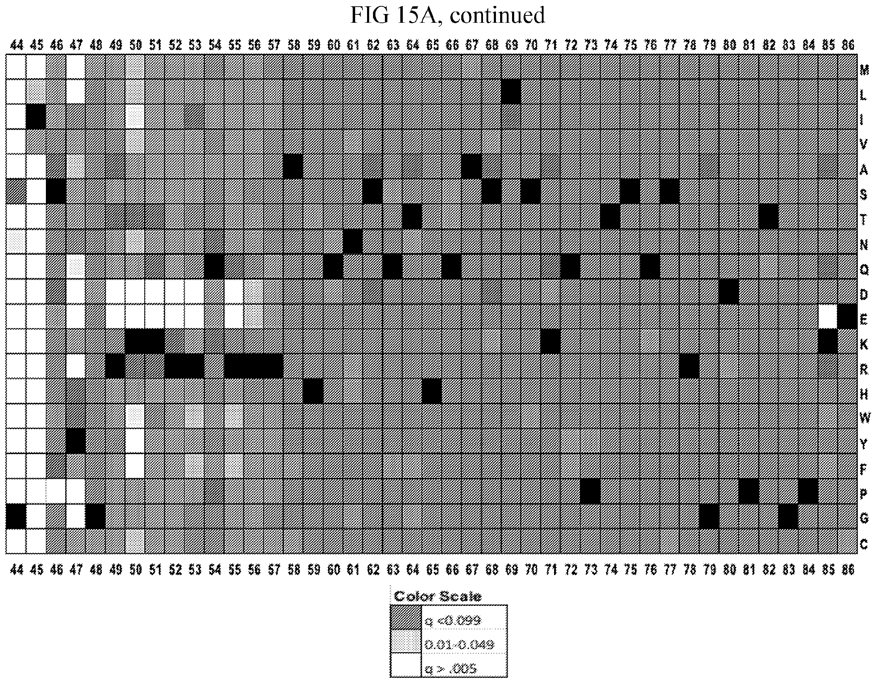

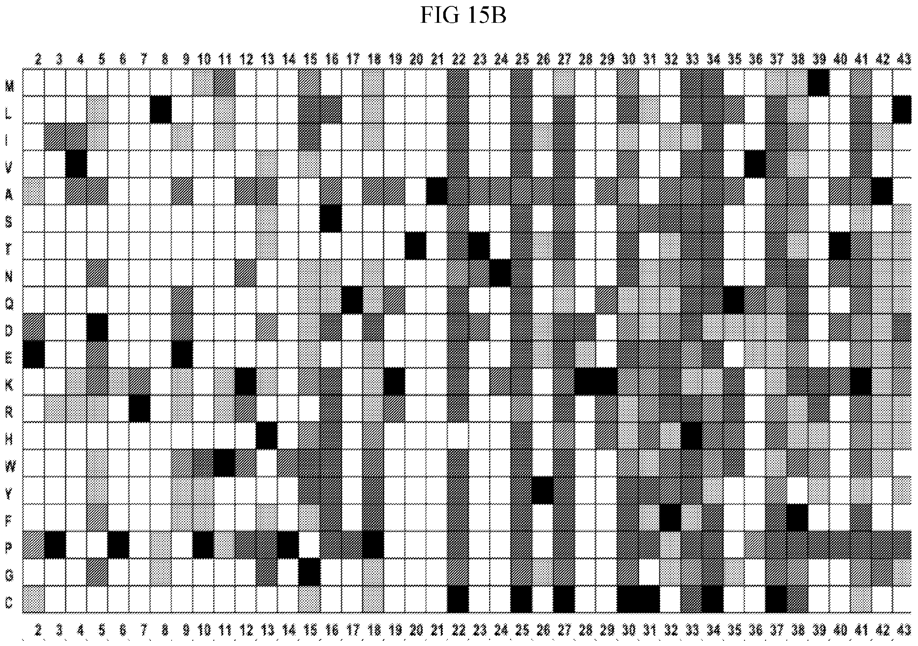

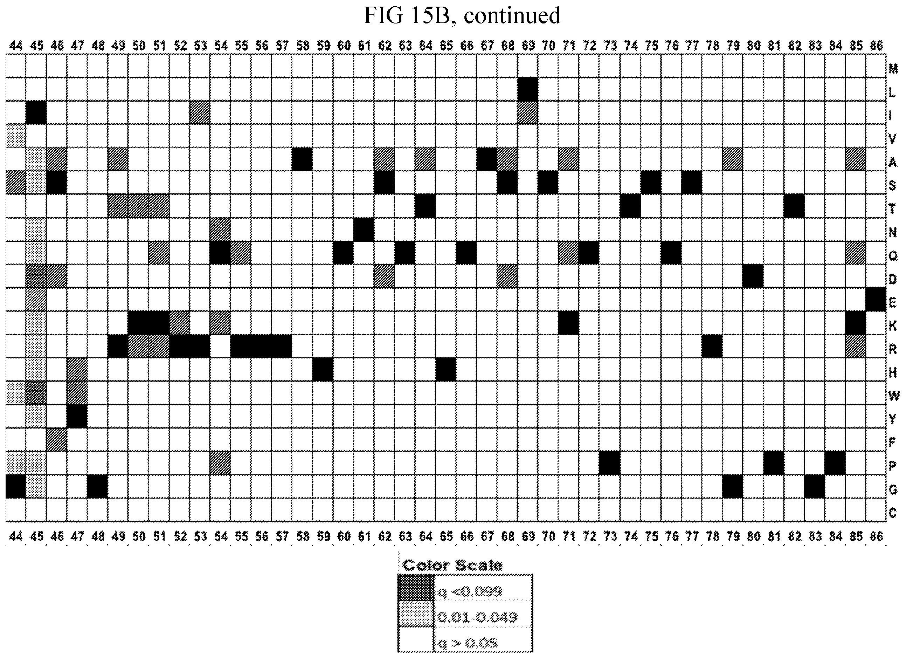

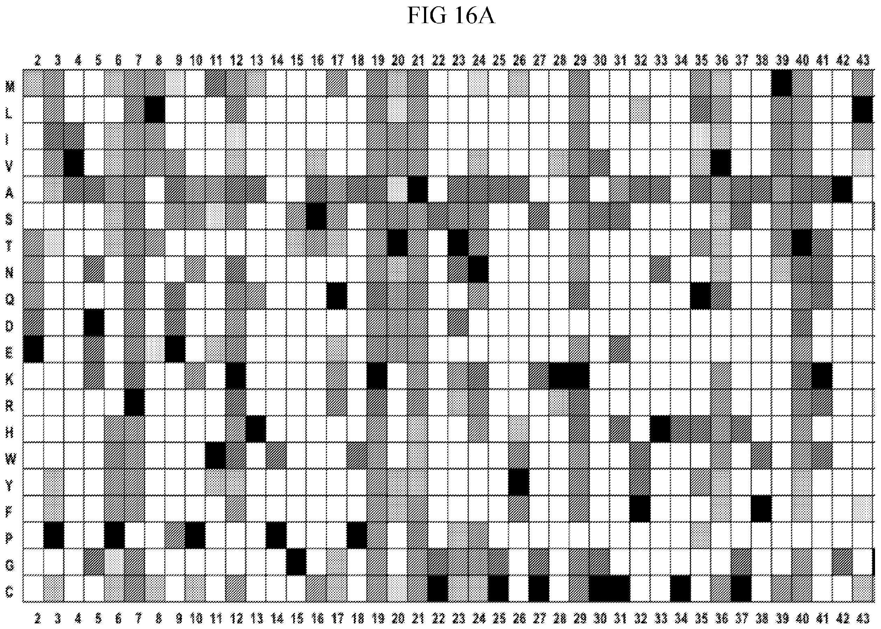

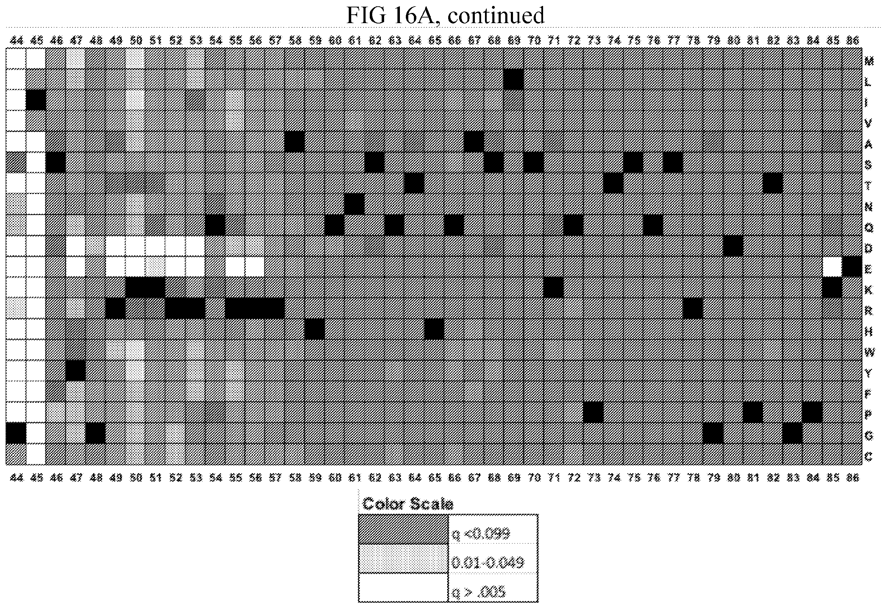

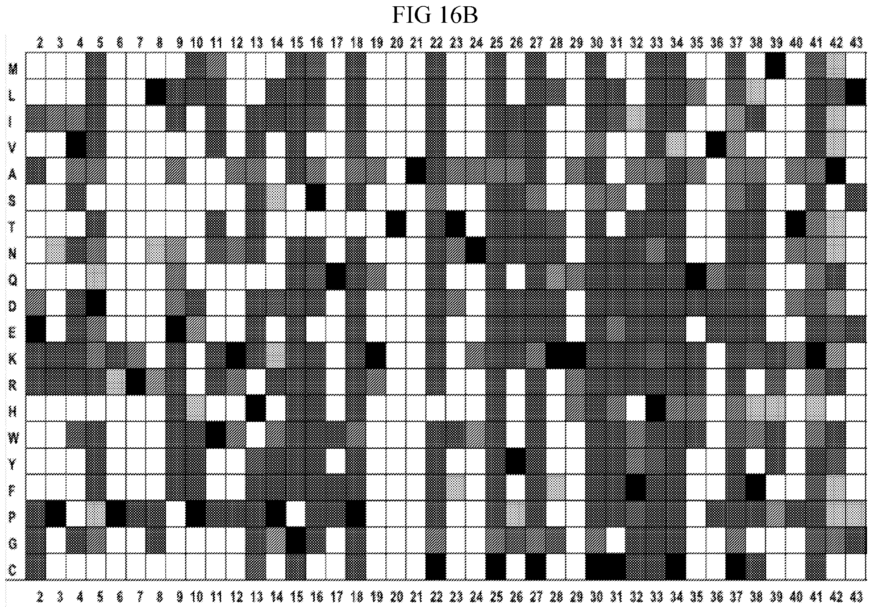

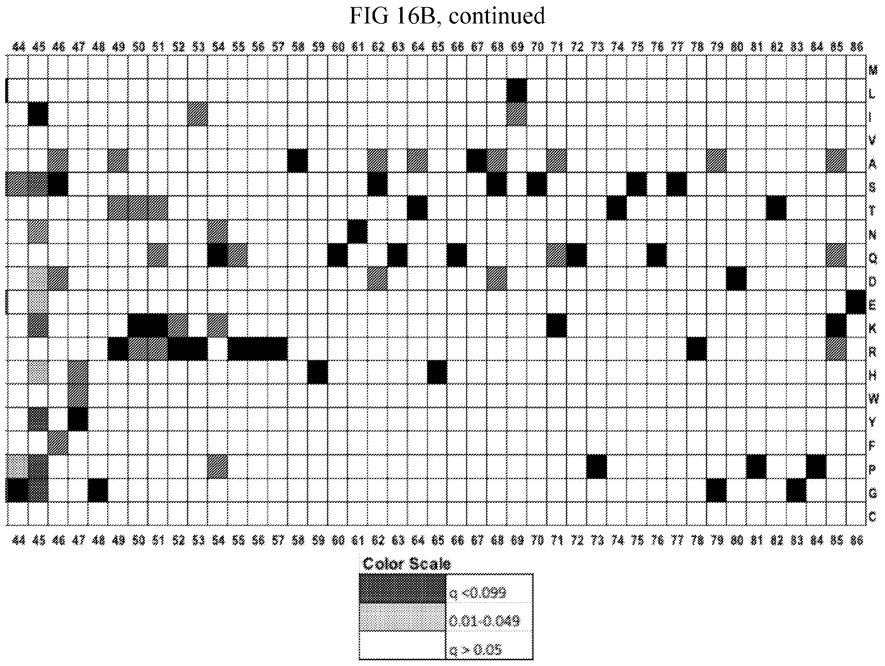

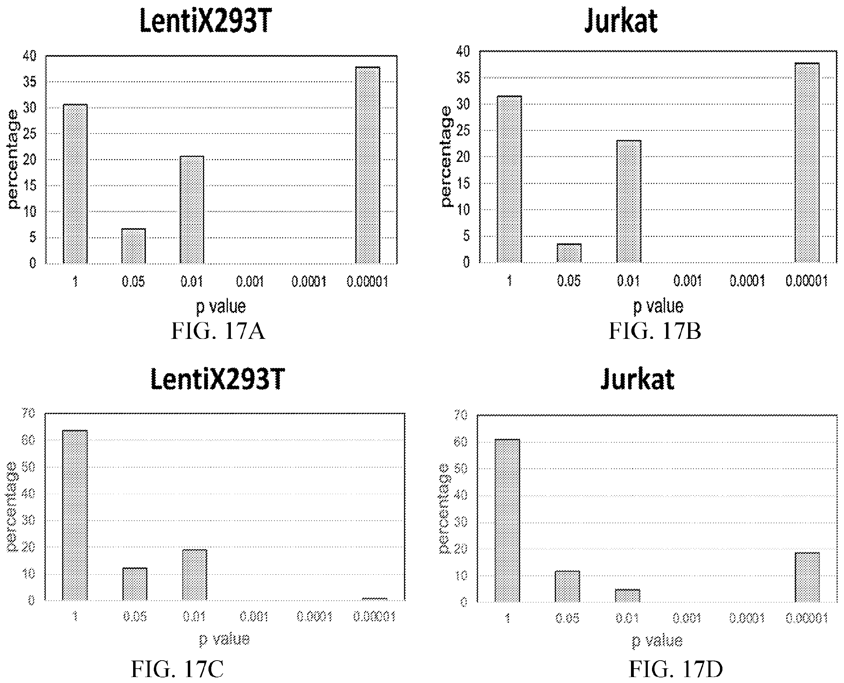

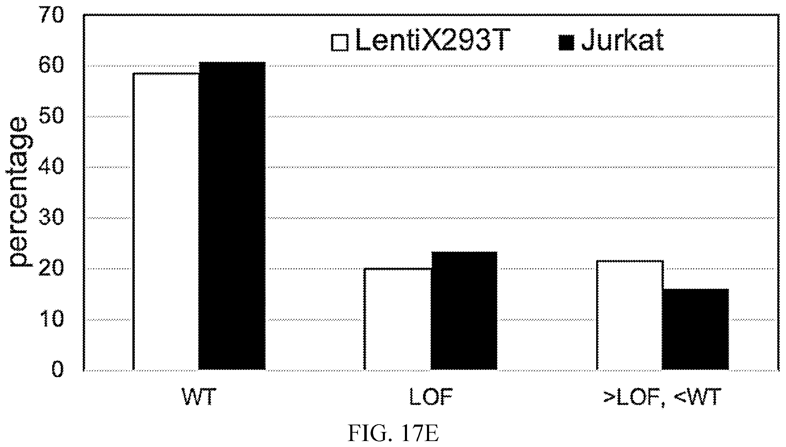

Each mutant's activity was then compared to that reported for sets of true positive mutants with established wild type activity and sets of true negative mutants with greatly reduced activity (e.g., see Figs. 15-17). In both cell lines, most substitutions have either wild type (58-60%, p < 0.05) or reduced activity (20-23 %, p < 0.05) (e.g., see Fig. 17). Approximately 26-22% of mutants have neither WT or reduced activity and are moderately inhibited.

### Structure/function/tolerance impact of Tat mutants.

The saturation mutagenesis profile allows for an improved interpretation of mutation tolerance on secondary structure, post-translational modifications (PTM)s and protein-protein interactions (PPI)s on Tat activity. Based on the saturating mutagenesis experiment and its interpretations, expansion of Structure/function to structure/function/tolerance, adding the latter term to reflect what amino acids can be tolerated to preserve structure/function.

How the secondary structure for each Tat position related to mutations can be examine. Tat is mostly random coil with some helix and turns. Mutations were well tolerated in the first turn, but not in the second and third turns (e.g., see Fig. 2A). The only mutations in the first turn (R7, L8) with low activity are R7P, L8P, and L8G The. second turn starting at K28 has the sequence 28KKCCF32 (e.g., see Fig. 2A). No mutations at C30 are tolerated and only C31A and C31S with small amino acids substitutions retain activity, supporting steric hindrance restrictions of Φ and Ψ angles in turns. Only conservative large hydrophobic and some small aliphatic substitutions of F21 retain activity. Mutations in the third turn (K41, A42) are generally not tolerated with only reduced activity for A42G and A42C. Scattered mutations in the helices have reduced activities and the random coil regions, in general tending to be more tolerant of mutations, especially at the C-terminus. Notably, no substitutions are tolerated at K41 or in six C residues in the Cys-rich domain. C31 tolerates substitutions of S, T, or small aliphatic amino acids and C31S was previously known to be active and a natural variant in clade C Tat proteins.

Studies then examined if mutation of any of the residues with PTMs impacted Tat activity (e.g., see FIG. 2A). Tat has 18 reported PTMs of five different types. 19,20 Mutations of single PTM positions do not impact transcriptional activity when mutated to other amino acids or likely impacted structure or other functions when all mutations of a position are considered. Tat is ADP ribosylated at E2 and E9 and several mutations at these positions that cannot be ADP ribosylated retained activity. Methylation of R7 is only blocked by R7P, which therefore likely is not due to PTM, but instead, due to constraints in folding of turn 1. S16 is phosphorylated, but even though T is tolerated and can be phosphorylated, other small amino acids such as C and A that cannot be phosphorylated still retain activity, indicate a non-essential role for this PTM. The K28 ubiquitylation site can tolerate substitutions that cannot be modified and is in turn 2, a secondary structure element that is prone to loss of activity when mutated.

Tat has known binding sites for about 20 other proteins (e.g., see FIG. 2A) of which nine have substitutions that inhibit transcriptional activity. Most interactions of these PPI sites are in a hotspot from residues 29-60.20,21 The most sensitive interactions are that of Cyclin T1 and Importinα. CyclinT1 binds15 amino acids in Tat, mostly in the Core region; 13 have at least one mutant that blocks Tat transactivation, and CyclinT1 is a key protein for recruiting RNA polymerase. 22,23 The Importinα interaction site (50KKRRQRRRAHQ60) is not very sensitive to single substitutions, although acid substitution from 50-56 mildly impaired activity, although this also could be through the overlapping with the RNApol2 binding site as well. The robustness of this site (52RRQRRRA57) to tolerate all substitutions, likely reflects robustness for key Importinα and CyclinT1/CDK9 complex recruitment. 22,24-27 The RNApol2 site also overlaps with EGR, P53, CA150 and 11S proteosome binding sites. There is much to be learned about Tat PPIs when considering this data. This region is also of interest based on the truncation mutants in the high-throughput cellular molecular function assay system and method.

The activities of 78 truncation mutants are measured from nonsense mutants across both cell lines. The mutants are introduced during oligonucleotide synthesis (e.g., see FIG. 2A). All 70 mutants with a truncation before amino acid 58 have no or little detectable activity, whereas the 8 mutants after amino acid 58 have nearly full activity. The perfect accuracy, PPV and NPV for this analysis is consistent with a protease cleavage site between residues 57-58, which is also an exon boundary, and previous observations support similar truncation tolerance in these regions.

However, almost any missense mutation is tolerated at S46-E86, which is not consistent with the truncation mutants tolerated up to R57. This region contains a nuclear localization motif, and binding sites for Importinα, P53, and EGR. The most likely explanation is that truncation of the nuclear localization signal blocks localization of Tat to the nucleus and its transcriptional activity. In the presence of any single point mutant, the Tat is still localized to the nucleus. This is supported by high-throughput cellular molecular function assay system and method results showing that mutation of all positively charged residues in the nuclear localization motif to negatively charged residues, reduced, but do not block the transcriptional activity of Tat. Indeed in papers that have examined the NLS, only double mutants in this region block nuclear localization, and the peptide is sufficient to localize other proteins to the nucleus.

The impact of mutation on activity by mapping transcriptional activity levels onto the 3D structure of Tat can be examined, which can be compared to the residue spatial positions, regions, and secondary structure of Tat (e.g., see FIGs 2A, 3A-3C). Ala Scanning mutagenesis is an accepted approach to identify positions important for different functions.32 Alanine scanning shows 18 LOF mutations scattered across the N-terminal half of the protein (e.g., see FIGs. 2A, 3D). Scanning with other amino acids such as Pro or Asp are more sensitive with 23-24 LOF mutations, Cys scanning is less sensitive with only 9 LOF positions that are a subset of Ala scanning, and Gly substitutions address flexibility (e.g., see FIGs. 2A, 3E, 3F, FIG. 18E, respectively). When these approaches are compared to the minimum, average, or maximum activity for all of the19 amino acid substitutions at each position or the positions that do not tolerate substitutions (e.g., see Figs. 2A, 3E-3G, Fig. 18F), information from the residue specificity in saturation mutagenesis is not completely captured in these types of 3D surface maps.

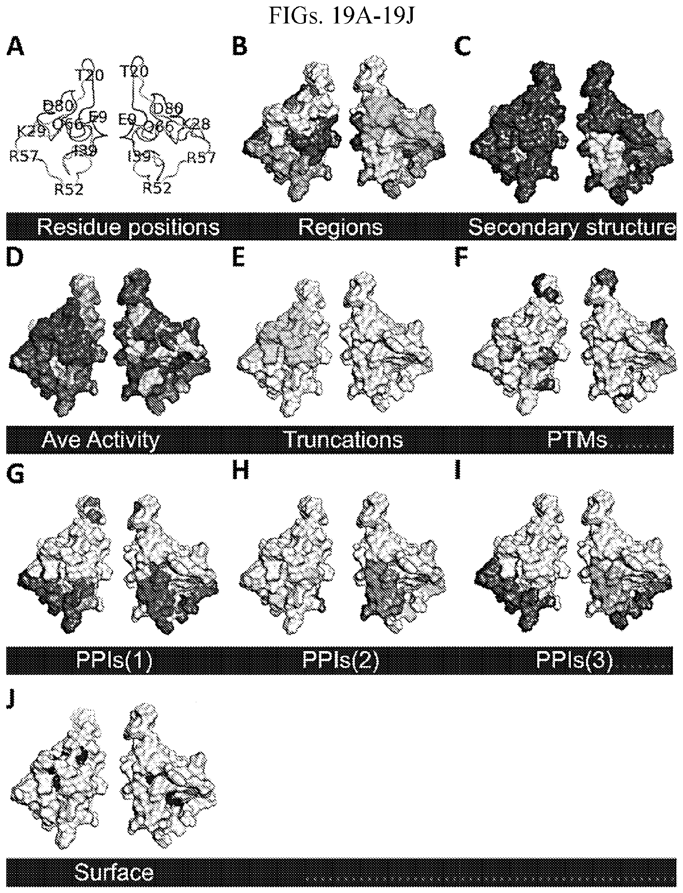

In some instances, a different approach that better summarizes the additional information gained from saturation mutagenesis, is to codify by separate Mathews Correlation Coefficients (MCC)s for groups with similar side chain physiochemical properties (small aliphatic, large hydrophobic, polar noncharged or charged, negatively charged, positively charged). This approach better segregates substitution tolerance for each position, whereas alanine scanning does not have this level of granularity. The heatmap with accuracy for classes of substitutions (e.g., see FIG. 19) shows that F32 only tolerates a large hydrophobic, G15 only tolerates a polar-noncharged, and C31 only tolerates amino acids with smaller volumes.

Surface plots of MCC heatmaps for different physiochemical properties show that there is little specificity for amino acid tolerance over most of the protein including residues S46-E86 which tolerate nearly all substitutions (e.g., see FIG. 3J-3I, FIGs. 18G-18L, 19). However, the specificity for different classes of substitutions is clustered in the Cys-Rich and Core regions (e.g. see FIGs. 2A-2B ,3B). These regions have seven residues that do not tolerate any substitution, have reduced activity in Ala, Pro, and Gly scanning mutagenesis, do not contained buried residues, and include key binding sites for CyclinT1, Importinβ and several other PPIs (e.g., see FIGs. 2, 3B,3D,3E, FIGs. 18E-18L, 19G-19J, S20). The new Physiochemical Accuracy Surface (PAS) plots and MCCs better identify residue tolerance of each position and their relative spatial locations, as well as surface accessibility (e.g., see FIG. 2, FIGs. 19J).

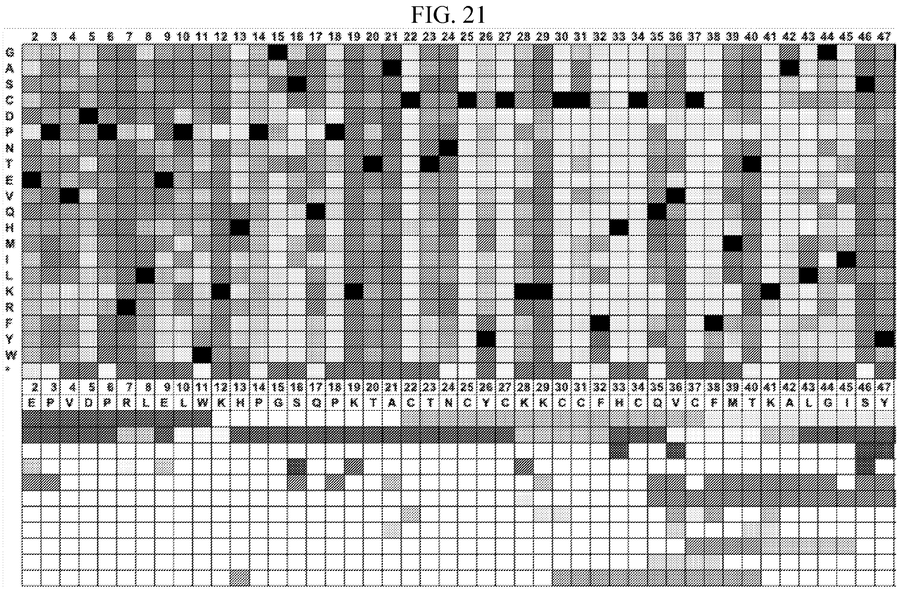

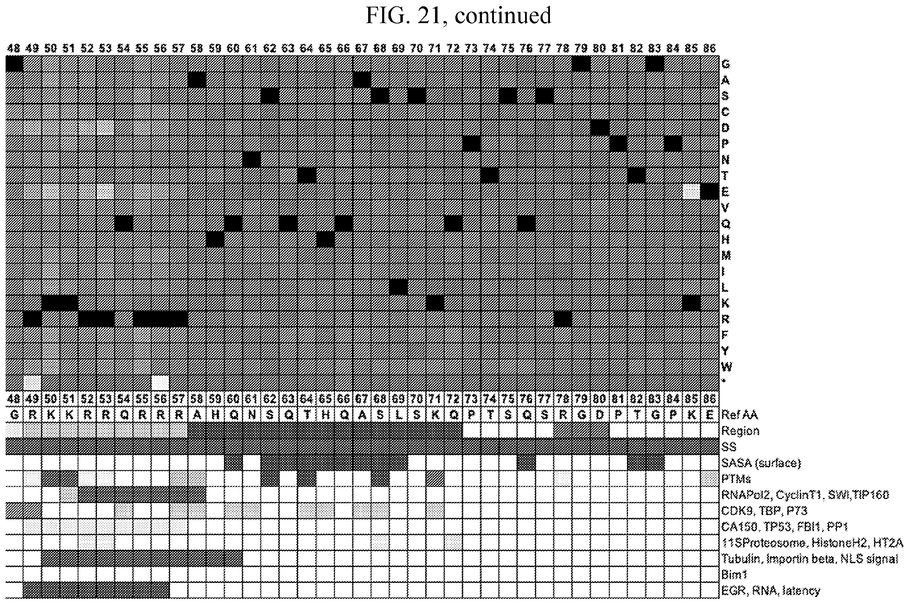

Likewise, side chain volume gradient indicates tolerance of amino acid substitutions for a sidechain volume (e.g., see FIG. 2A, FIG. 21). Positions Y26, F32, and F38 prefer large amino acids, positions E9, L10, G15, S16, T23, C31, M39, and A42 prefer small amino acids, and positions D5, C25, L43, and I45 favor medium sized amino acids. Overall, some positions do not tolerate substitutions, some positions tolerate substitutions that are tolerated driven by side chain volume, whereas a well-defined spatial region tolerance is driven by a combination of secondary structure and/or physiochemical properties of sidechains.

### EXPERIMENTAL EXAMPLES

The invention is now described with reference to the following Examples. These Examples are provided for the purpose of illustration only, and the invention is not limited to these Examples, but rather encompasses all variations that are evident as a result of the teachings provided herein.

The materials and methods employed in these experiments are now described.

### Cloning

The plasmid pLjm1_mcs are made by introducing compatible EcoRI, SalI, and AsiSI restriction enzyme sites in the pLjm1-Empty (Addgene) vector for cloning of the Tat variant library. Tat or mutant Tat encoding a C27S mutation are PCR amplified from pNL4-3 as a template with Q5^{®} High-Fidelity DNA Polymerase (New England Biolabs) and cloned into EcoRI/SalI digested pLjm1_mcs1. For generating a LentiX293T/LTR-GFP reporter cell line, a plasmid harboring LTR-GFP and blasticidin S resistance is constructed. The LTR-GFP cassette and Blasticidin S resistance (bsr) gene are amplified by PCR with pNL4-3, pEGFP and LentiCRISPR-v2 Blast as templates. LTR, GFP, and bsr amplicons are fused by inverse PCR using Q5^{®} High-Fidelity DNA Polymerase. The fused amplicons are cloned into pAAVS1-Puro-DNR (Origene) previously digested with SpeI and EcoRI.

### Generation of barcoded variant plasmid library

A double stranded (ds) DNA library containing HIV-1 Tat cDNAs with sequences for all the possible single amino acid mutant mutants (n =1634 Tat mutants) are synthesized by Twist Bioscience. The dsDNA from each well of 96-well plates are pooled and a single round of overlap PCR extension appended random 32mers oligonucleotides to the 3' untranslated region. The synthesized dsDNA library has a 3'-overhang sequence after the stop codon that overlaps with the 5' overhang sequence upstream of the 32mers random oligonucleotide sequence. The pooled ds DNA library and the random oligomer are mixed in 1:10 molar ratio, denatured, and annealed. Hybridized DNA is extended with the Q5^{®} High-Fidelity DNA Polymerase (New England Biolabs) for one cycle of PCR. The 50 µl of PCR reaction mix is then treated with 2 µl of Exonuclease I (New England Biolabs), incubated at 37°C for 15 min, and DNA is purified by PCR cleanup kit (Macherey-Nagel).

The purified DNA is digested with EcoRI-HF (New England Biolabs) and AsiSI (New England Biolabs) for 3 h at 37°C and ligated into EcoRI-HF/AsiSI digested pLjm1_mcs plasmid (molar ratio vector: insert = 1:3) with electroligase (New England Biolabs). Ligation reactions (12) are pooled, purified with a PCR cleanup kit, and drop dialyzed on MF-Millipore^{®} Membrane Filter, 0.025 µm pore size (Millipore Sigma). The purified ligation reaction mixture is electroporated into E. cloni 10G ELITE electrocompetent cells (Lucigen), plated on prewarmed LB ampicillin plates, and incubated for 18 h at 37oC. Transformants were scrapped and plasmid library from the pooled cell suspension is isolated using EndoFree Plasmid Mega kit (Qiagen).

### Production and tittering of lentiviral libraries

Lentiviral libraries are produced in LentiX293T cells (Takara). Approximately 3 million LentiX293T cells are seeded in 100 mm petri dish and grown in 10 ml complete DMEM media [(DMEM+10% Fetal Calf serum), Gibco] for 24 h. Plasmids pLjm1_Twist Tat Library (8.5 µg); pMDLG/pRRE (7.6 µg); pRSV/pRev (4.0 µg); pMD2.G (4.0 µg) are diluted to a final volume to 613 µl in a 15 ml conical tube. CaCl2 (87 µl of 2M) are added to plasmid mixture. 2XHBS (700 µl) are added dropwise to the above transfection mix with gentle stirring in a circular motion. The transfection mix is incubated for 15 min and added dropwise to the cells in a 100 mm petri dish. The cells are incubated at 37°C for 12 h in a CO2 incubator at 37°C with a 5% CO2 atmosphere. Post-transfection (12 h), the calcium phosphate-containing medium is replaced with 7 ml complete media (DMEM+10%FBS) and incubated for 48 h in CO2 incubator at 37°C with a 5% CO2 atmosphere. Spent media from confluent transfected LentiX293T cells is filtered through a 0.45 µm Uniflow syringe filter (Cytiva Whatman). Aliquots of the filtered spent media with the lentivirus (100 µl to 5 ml) are stored in at -80°C.

Lentiviral vectors for specific clones are produced in LentiX293T cells. Briefly, the 0.6 million LentiX293T cells are seeded in a well of a 6-well plate. After 24 hours, cells are co-transfected with pLjml-mcs, pLjm1-Tat, or pLjm1-TatC27S (1 µg); pMDLG/pRRE (Addgene, 1.0 µg), and pRsv-Rev and pMD2.G (Addgene, 0.5 µg) transfecting with Lipofectamine LTX (Invitrogen) at a 1:3 ratio [DNA(µg): Transfection reagent(µl)]. After 6 h of incubation, media is replaced, and cells are cultured in complete media for an additional 48 h. Cell supernatants are collected, filtered through a 0.45 µm syringe filter (Millipore), and stored in -80 freezer.

Lentiviruses are titered by seeding 10,000 cells/well in 96 well plate and culturing in 200 µl of complete DMEM media (DMEM+10% FBS). After 24 h, 100 µl of serial dilutions of lentivirus are added after removing majority of the spent media from the wells and incubated 4 h. Complete DMEM media (100 µl) are added and incubated 24 h. Spent media (100 µl) is removed, replaced with DMEM media containing puromycin (Invitrogen, 1.5 µg/ml final concentration), and incubated for 96-120 h. The cells are inspected for viability under the microscope and colonies are counted to calculate the infectious unit/ml.

### Generation of LentiX293T/LTR-GFP and Jurkat/LTR-GFP reporter cell lines

LentiX293T cells (0.6 million) are seeded in the well of a 6-well plate and grown in 3 ml of complete DMEM media. A GFP reporter plasmid (1.5 µg) carrying LTR-GFP and the blasticidin S-resistance (BSR) gene is transfected in LentiX293T cells and incubated for 48 h. Transfected cells are selected for blasticidin S [(5 µg/ml), Invitrogen] resistance for 14 days, exchanging DMEM media with the poison every 3 days. Cells are trypsinized and 100,000 cells are serially diluted in 96-well plates. After 14 days of incubation, single colonies are screened after expansion.

For confirming lentiviral integration, gDNA is isolated Tat amplicons are subcloned, and sequenced. Tat transcriptional activity is measured in a subculture of each clonal cell line. Cells culture in 96-well plate are transfected with 50 ng of wtTat expression vector and cultured for 48 h. Transactivation-induced GFP expression is evaluated by Nikon TE2000E epifluorescence microscopy. The clonal reporter cell lines are propagated and stored at -80°C.

### Stable cell libraries and cell lines

LentiX293T/LTR-GFP cells (33 million) are transduced with the Tat variant lentiviral library at a multiplicity of infection (MOI) of 0.1. After 24 h of infection, cells are cultured and maintained in complete DMEM media supplemented with puromycin (1.5 µg/ml). After 5 days, confluent cells are harvested, counted, and washed once with 1X PBS before fixing and isolating gDNA for NGS of the Tat amplicon.

Jurkat/LTR-GFP cells (90 million cells) are seeded and transduced with 0.1 MOI of lentiviral library for 4 hours. One day after transduction, the cells are selected for viral survival in RPMI 1640 media [(RPMI 1640+10% FBS), Gibco] supplemented with puromycin (1 µg/ml). After 5 days, the cells are counted, washed with 1X PBS, and fixed for flow sorting and subsequent isolation of gDNA.

For performance evaluation of the high-throughput cellular molecular function assay system and method, 18 random mutants of Tat, as well as empty vector and wtTat are stably expressed in LentiX293T/LTR-GFP cells. Approximately 0.15 million cells are seeded in a well of a 24 well plate and incubated for 24 h. Cells are transduced with lentivirus and selected and maintained in complete DMEM media with puromycin (1.5 µg/ml) for 96 h. Cells re harvested and analyzed by flow cytometry to assess for LTR transactivated GFP expression. The same stable cell lines are created in Jurkat/LTR-GFP cells. Selected clones for empty vector, wtTat, and Tat C27S are stored at -80°C.

### Flow sorting of cells and deep sequencing

One fourth of the LentiX293T/ LTR-GFP and one tenth of the Jurkat/LTR-GFP cells are harvested, gDNA isolated using Qiagen DNeasy Blood & Tissue Kit, and sequenced to evaluate library representation before Flow Sorting. The remaining cells are fixed in 2% paraformaldehyde/PBS for 10 minutes, washed twice with 1X PBS and resuspended in 1X PBS for analysis by flow sorting (Sony 800S Cell sorter). Cells sort into three bins of GFP signal intensity (low-GFP, mid-GFP and high-GFP) gated with threshold determined for cells stably expressing wt-Tat for maximal transactivation of LTR-GFP, and cells stable expressing a Tat C27S mutant or empty vector for low background of basal transactivation of LTR-GFP.

For deep sequencing, primers are designed to flank the Tat targeted region from gDNA and incorporate the NGS sequencing adaptors. gDNA is amplified by PCR with NEBNext Q5 Hot Start HiFi PCR Master Mix. The PCR protocol denatured strands at 98 °C for 30 sec only in the first cycle followed by: denaturation at 98 °C for 10 s, annealing at 58 °C for 15 s, elongation at 72 °C for 30 s, and a final elongation for 2 min. NGS libraries for each sample category used 10 NGS library forward primers and 1 NGS library reverse primer. The forward primers are common for all the sample categories and the reverse primer being unique for each sample. The Tat amplicons are pooled and 20 µl of the sample is purified by gel extraction with Ampure-XP beads (Beckman Coulter). All the samples are pooled and sequenced with a Novaseq 6000 sequencing platform. This SP flow cell produces approximately 2x250 bp paired-end reads. 18 samples were sequenced (synthetic dsDNA Tat variant library, plasmid library, selected cell libraries in LentiX293T and Jurkat cells (in duplicate), Flow sorted low-GFP, mid-GFP, and high GFP cells for each cell line (in duplicate).

### Processing NGS data with a bioinformatics pipeline

Paired-end reads were processed with a multistep bioinformatic pipeline BaseSpace and resulting reads in bcl files were converted into FASTQ files with BCL2FASTQ, read quality is assessed with FASTQC (e.g., see Fig. 5).34 Paired end reads for all samples are merged with FLASH to build complete Tat contigs.35 Contigs were quality trimmed with Trimmomatic.36 Adapters are trimmed, and 32 nt barcodes are isolated with CutAdapt and Starcode is used to group bacodes.37,38 The sequence reads are demultiplexed into subsets of read sequences for each cell clone based on unique barcodes with a custom Python script that processes the output of Starcode. Resulting reads are then aligned to the Tat cDNA with BWA MEM.39 The BAM file with nucleotide variants are called for each subset of Tat contigs (cell clones) and output as a VCF file with BCFtools (mpileup).40 Custom Python scripts are used to identify the amino acid substation for the VCFs, the number for reads for each barcode in each sample, and the barcodes groups for cells with the same amino acid substitutions. The PyVCF library is used in scripts that gathered the information for each variant from the VCF files.41 Read counts and read depths for each barcode and each amino acid substitution in each sample are normalized to the number of reads/million and activity is measured by the percentage of GFP+ reads for each barcode and each mutant.

### Data analysis, statistics, and figure preparation

Statistics are calculated for each mutation. In some instances, there are n cell lines (biological replicates) and each cell line has m technical replicates. For each barcode (group) in a sample, we calculate the percentage of the number of reads in the GFP+ group vs the total number of reads in both GFP+ and GFP- groups, denoted as h ratio (hE[0,1]). In some instances, a high h percentage for wild type, while a low h percentage suggests a mutant. Then for each mutant, calculate the averaged h ratio for all the barcodes assigned to the same mutant, denoted as a mutant level summary score. In some instances, use a one sample t-test to evaluate 1) whether the mutant has a significantly different number of reads in the GFP+ group compared with the GFP- group within a technical replicate, and 2) whether the mutant has a significantly different number of reads in the GFP+ group compared with the GFP- group among different cell lines based on biological replicates (null hypothesis H0: h =0.5).

In some instances, classify mutants with high h percentage as wild type and a low h percentage as a LOF mutant. To estimate type I error for the classification, in some instances compile a list of true mutants with wild type transcriptional activity and true LOF mutants with low activity. Then fit their h percentages with a beta distribution as the null distribution. Specifically, for the wild type detection, in some instances, use the true mutant as the null, and vice versus, for the mutant detection, use the wild type as the null. Moment estimators are used for estimating the model parameters. The p values for different cell lines are combined using Fisher's method into a global test p value.

Performance metrics of accuracy, sensitivity, specificity, positive predictive value and negative value are based upon standard formulas.

Figures can be prepared with PowerPoint, Excel, FlowJo, and Pymol. Bin, Bar, and Pie plots, as well as saturating mutagenesis heatmaps generated with Excel. Values for saturating mutagenesis heatmaps and 3D surfaces plots can be generated with custom python scripts. 3D surface plots for the amino acid tolerance at each position represented accuracy of physiochemical properties as gradients from blue to white to magenta, with magenta being the highest accuracy. Accuracy is a standard formula and is calculated for groups of amino acids with similar physiochemical properties.14 Solvent accessible surface area (SASA) is calculate for the Tat structure (1TIV) with the Accessible Surface Area and Accessibility Tool.42 Residues are considered buried if less than 10% of surface area is exposed to solvent.

The MCC formula is calculated with the following data definitions for large hydrophobic amino acids, at a position in Tat as an example: If either Phe, Tyr, or Trp have > 50% activity they are true positives and if the other amino acids have <50% activity they are true negatives. If either Phe, Tyr, or Trp have <50% activity they are false positives and if the other amino acids have >50% activity they are false negatives. Also consider the wild type amino acid to be a true positive when it is in the physiochemical group, and as a true negative when it is not. The MCC captures the tolerance for types of amino acids at each position and when mapped the surface of the 3D structure, is a new visual mining approach to reveal the spatial relationships of amino acids tolerances and their relevance to other Tat functions.

**Table 1. Summary pipeline statistics for next generation sequencing of high-throughput cellular molecular function assay system and method libraries.**

| Step | Reads (start) | % yield (by step) | Barcode | Mutants | Runtime |
|---|---|---|---|---|---|
| Fastqc | 368,408,071 | 92.2 | - | - | 226 min |
| Flash | 327,536,831 | 88.9 | - | - | 210 min |
| Trimmomatic | 323,651,918 | 98.8 | - | - | 175 min |
| Adapter Trimming | 323,163,108 | 99.9 | - | - | 86 min |
| Barcode Extraction | 323,163,108 | 100 | - | - | 60 min |
| Barcode Grouping | 294,692,904 | 91.2 | - | - | 30 min |
| Demultiplexing | 294,692,904 | 100 | - | - | 90 min |
| Variant Calling | 164,852,217 | 55.9 | Total: 561,000 | 1,774 | 9.5 days |
| | | | Unique:180,091 | | |
| Filtering - 2.5 rpm | - | - | Unique: 179,763 | 1,685 | 1 sec |

**Table 2. Summary performance statistics for next generation sequencing of high-throughput cellular molecular function assay system and method libraries.**

| Cells | Validation Method | Accuracy | Sensitivity | Specificity | PPV | NPV |
|---|---|---|---|---|---|---|
| LentiX293T | Benchmark data | 0.93 | 0.95 | 0.89 | 0.95 | 0.89 |
| LentiX293T | Nonsense mutations | 1.0 | 1.0 | 1.0 | 1.0 | 1.0 |
| LentiX293T | Validated clones | 0.94 | 0.75 | 1.0 | 1.0 | 0.92 |
| Jurkat | Benchmark data | 0.94 | 0.94 | 0.92 | 0.96 | 0.89 |
| Jurkat | Nonsense mutations | 0.91 | 0.90 | 0.93 | 0.96 | 0.82 |
| Jurkat | Validated clones | 0.87 | 0.50 | 1.0 | 1.0 | 0.86 |

**Table 3 : Variant calling sub-steps.**

| Step | Group Function | Program |
|---|---|---|
| 1. | BWA Indexing | BWA index |
| 2. | BWA Alignment | BWA mem |
| 3. | Sam to Bam | samtools view |
| 4. | Bam Sorting | samtools sort |
| 5. | Bam Indexing | samtools index |
| 6. | Pileup | samtools pileup |
| 7. | Variant Calling | bcftools call |

### Example 1: A bioinformatic pipeline to analyze a library of tat mutants

### Expected Amplicon

Sequencing was done through Illumina paired-ended sequencing. A forward and reverse read, each of size 250 base pairs, was utilized to capture our expected amplicon of size of around 465 base pairs. Within each of our reads is the tat encoding region (with 1 mutation introduced by the library,) as well as the 32 base pair unique molecular identifier used as a barcode. These parts of interest are extracted and used for our statistics and interpretation based on the quantification of barcode to variant mappings found in each of the flow sorted pools.

### Bioinformatic Methods

Raw data (Files 6-39) were analyzed through a custom bioinformatic pipeline to extract the tat encoding region and barcode. Each file was individually input to Fastqc to measure read quality estimates. The default parameters were used, and each file was deemed suitable to proceed forward with minor quality filtration downstream. Forward and reverse reads were to be fused to allow downstream analysis. This was accomplished using FLASH. Additional parameters used for fusion were minimum overlap length of 20, maximum overlap length of 45, and mismatch ratio of 0.15. After the forward and reverse reads were fused, Trimmomatic was used to perform quality filtration. Each file was filtered such that reads were required a minimum of 365 base pairs (BPs). Additionally, reads were removed if they contained a consecutive series of 4 BPs where average PHRED score across the four BPs drops below 16. Next, Cutadapt was used to remove adapter sequences. Reads were trimmed to just the Tat encoding region, a small 3' extension, and the 32 BP barcode. This was done by performing a linked adapter trim, specifying the parameter "-a AATTC...GCGATCGC" (SEQ ID NO: 1), which indicates to Cutadapt to identify a series of BPs surrounded by the specified sequences, and extract it.

After adapter trimming, the 32 BP barcode must be isolated to allow barcode grouping. This is accomplished using Cutadapt. Specifying a 5' adapter as follows "-g GATCTG", causes Cutadapt to trim away the rest of the reads, and yields the 32 BP barcodes. Once the barcodes are extracted, to account for any naturally occurring changes to the barcode that may arise during the cell cycle, the barcodes must be grouped. All barcodes extracted from the 17 sort files were pooled, and Levenshtein grouped with a distance of two using Starcode. Utilizing the ID retaining feature of Starcode, reads were able to be demultiplexed based on the barcode group they were assigned, as well as which of the original sort files the read came from. During demultiplexing, a strict size enforcement of 32 was enacted; barcode groups that were larger or smaller than 32 BPs were removed. The end result is many directories generated and named after each identified barcode group. Each of these directories contains up to 17 files which correspond to the reads associated with that barcode group, and its flow sort results. For example, barcode group AGACGTACCAACAAAAGACAATGACAAAAAGG (SEQ ID NO: 2) was associated with 1,447 reads across the flow sorted files. 34 reads corresponded to the replicate 1, GFP high sort, on cell line 293T; 25 reads corresponding to the replicate 1, GFP low sort, cell line 293T; etc.

Once demultiplexing has taken place, variants must be identified across all barcode groups. The variant calling pipeline starts by utilizing Burrow's Wheeler Aligner (BWA). The wildtype *TAT* sequence was BWA indexed and used as a reference for the rest of the variant calling. Each flow sorted file within each barcode group directory was then individually BWA aligned to the indexed reference file, using BWA *mem.* After alignment, the files were put through a series of processes in the Samtools package. The sam files generated from BWA mem were transformed into bam files, sorted, indexed, and pileuped through utilizing the Samtools *view*, *sort, index,* and *pileup* functions respectively. Lastly BCFtools' *call* function resulted in a VCF file for variant interpretation.

For each barcode group, all 17 VCFs were compared to ensure a valid variant calling was performed. For validation, All 17 VCFs should conclude on a particular codon substitution, as designed in the experiment. Once validated, read counts supporting the codon substitution were extracted from the flow sorted files. Read counts were normalized to reads per million (RPM) and a comparison of the distribution of where these read counts land in the flow sorted samples across all barcodes for a particular variant yields the resulting activity measures as shown in data file 1. Placing these scores into a heatmap and showing known post translational modification sites of tat results in FIG 3. The number of barcodes associated with each mutant was quantified as shown in figure 4. Reads per mutant were also quantified and are shown in FIG 5. Matthew's correlation coefficient was calculated for each amino acid's physicochemical group and is recorded in FIG 14.

For validation, a mutant was considered active if 50% or more of the normalized reads associated with the mutant were from the high bin of the flow sorted pools. Barcodes were only considered valid if there were at least 2.5 RPM. The distribution of the mutant's activity ranges is shown in figure 2. Validation was done in several ways. The first validation method was to compare against 164 findings in the literature recorded in data file 2. Validation was also done by comparing the activities of the found nonsense mutants recorded in data file 3. The GigaAssay was also validated by comparing the activity against previously measured mutants. 18 mutants' activities were recorded individually as shown in FIG 6, then compared against how they performed as part of the library. Mutant activities were compared across cell lines to ensure valid measures, shown in FIG 7.

Statistical analysis was done through two models. The first aimed to capture mutants whose activity was wildtype-like or loss of function (LOF). For each barcode in a sample, the percentage of reads in the high bin out of reads in the high and low bin was calculated, and is denoted as *h* ratio (*h* ∈ [0,1]). A high *h* percentage resembles wild type, while a low *h* percentage suggests a mutant. For each mutant, we calculate the averaged *h* ratio for all the barcodes assigned to the same mutant, denoted as a mutant level summary score. A one sample t-test was used to evaluate 1) whether the mutant has a significantly different number of reads in the high bin compared with the low bin within a technical replicate, and 2) whether the mutant has a significantly different number of reads in the high bin compared with the low bin among different cell lines based on biological replicates (null hypothesis H₀: *h* =0.5).

In addition to the t-test comparing the high bin ratio among the mutants, a test was devised to perform an association between the genotype (LOF/wildtype) and GFP expression (binary variable high bin or low bin.) A mixed effect logistic regression was used, with random intercepts for barcodes and replicates to model the nested structure in our experimental design. For the wildtype control populations, we used the cells with no mutant calls (sequences identical to the reference). Each mutant was compared against the common wildtype control population. The model M1 with genotype included as fixed effects was compared to a null model M0 without genotype in a likelihood ratio test (LRT). Similar to Genome-Wide Association Studies (GWAS), a significant result indicates that the LOF/wildtype is associated with the percentage of high bin cells. For mutants where the model fit was singular, we simplified the model by dropping the random effects. p-values were false discovery rate (FDR)-adjusted using Storey's q-values.

### Enumerated Embodiments

The following enumerated embodiments are provided, the numbering of which is not to be construed as designating levels of importance.

Embodiment 1 provides a method of analyzing results of a high-throughput assay system for molecular functions and cell processes, the method comprising:
generation of a plasmid cDNA library for a gene by oligonucleotide synthesis or other mutagenesis methods; the expressed gene of interest encodes a combinatorial set of one or more amino acid substitutions; each cDNA molecule in the library also comprises a unique and random barcode;
conversion of the plasmid cDNA library into a lentiviral library or another type of library for delivery to mammalian cells;
engineering of a human or mammalian cell line to encode a reporter assay to measure a specific molecular function or cell process;
providing a population of cells, each cell comprising a gene of interest and an engineered reporter element
sorting the population of cells into a plurality of pools based on a change in a measurable property of the reporter element in response to the activity of the gene of interest;
purifying the genomic DNA from each pool and performing targeted next-generation sequencing for the integrated gene cDNA and barcode to generate a plurality of paired end reads for each cell-sorted pool;
subjecting the paired end reads to a quality control step;
fusing the paired end reads at the reverse complement overlap region to build a complete contig for the cDNA and barcode;
   trimming the reads and extracting the barcode sequences;
grouping the barcodes from all sorted pools and performing demultiplexing to create barcode groups of identical or closely-related barcodes;
performing variant calling on the barcode groups to one variant call format file; and
processing the variant call format file to associate amino acid substitutions encoded by each cDNA with a barcode group, determine read frequencies for each barcode, barcode group, and cDNAs encoding the same amino acid substitution(s);
   and
   calculate an activity measurement to for each barcode, barcode group, and cDNAs encoding the same amino acid substitution(s) based on its distribution among the flow-sorted pools to determine the affect each mutation in the protein of interest has on its measured function; comparing the distributions of the activities for the barcode and cell groups for each mutant to that of another set of cell groups where the cell group has wild type cDNAs without mutants, mutant cDNAs that are established loss of function mutants, or other classes of mutants; the comparison of the groups are with a statistical model to test specific hypotheses regarding the activity of each mutant.

Embodiment 2 provides the method of embodiment 1, wherein the gene of interest is a transcription factor.

Embodiment 3 provides the method of embodiment 1, wherein the reporter element encodes a promoter element that drives expression of a cDNA encoding a fluorescent protein.

Embodiment 4 provides the method of embodiment 3, wherein the fluorescent protein is GFP.

Embodiment 5 provides the method of embodiment 1, wherein the variant calling step further comprises:
performing Burrows-Wheeler indexing and alignment to a wild type reference sequence for each group of related barcodes to generate a sequence alignment and map (SAM) file;
converting the SAM file to a binary alignment and map (BAM) file;
sorting and indexing the contents of the BAM file;
aligning the contents of the BAM file to generate alignments of the variants to the wildtype; and
generating a variant call file from the alignments.

Embodiment 6 provides the method of embodiment 1, wherein the barcode comprises 32 or more nucleotides.

Embodiment 7 provides the method of embodiment 1, wherein the cells are mammalian cells.

Embodiment 8 provides the method of embodiment 1, wherein the cells are an immortalized cell line.

Embodiment 9 provides the method of embodiment 1, wherein the cell sorting is accomplished by flow cytometry.

Embodiment 10 provides the method according to embodiment 1, wherein the population of cells comprises an average of hundreds of separately randomly barcoded cDNA molecules assigned to each cell group for each mutant.

Embodiment 11 provides the method according to embodiment 1, wherein each mutant has a read depth of about 2,000X to about 90,000X sequencing coverage.

Embodiment 12 provides the method according to embodiment 1, further comprising validating the method by comparing the activity of a subset of mutants analyzed by the method to previously determined results.

Embodiment 13 provides the method according to embodiment 1, further comprising validating the method by comparing true negatives as determined by the method to true negatives determined by an independent method.

Embodiment 14 provides the method according to embodiment 1, further comprising validating the method by comparing the activity of a subset of mutants analyzed by the method to independent testing of a set of separate clones.

Embodiment 15 provides the method according to embodiment 1, further comprising validating the method by comparing method results among different samples.

Embodiment 16 provides the method according to embodiment 1, further comprising validating the method by comparing method results in two different cell lines.

Embodiment 17 provides the method according to embodiment 1, wherein the statistical significance of mutants analyzed in the method has a median p value of p < 9 x 10⁻²⁰.

### Other Embodiments

The foregoing description, for purpose of explanation, has been described with reference to specific embodiments. However, the illustrative discussions above are not intended to be exhaustive or to limit the invention to the precise forms disclosed. Many modifications and variations are possible in view of the above teachings. The embodiments were chosen and described in order to best explain the principles of the present systems and methods and their practical applications, to thereby enable others skilled in the art to best utilize the present systems and methods and various embodiments with various modifications as may be suited to the particular use contemplated.

## Claims

1. A method of determining an effect of a variant of a gene of interest on a molecular function or cell process in a eukaryotic cell, the method comprising:
generating a library comprising a plurality of nucleic acid molecules encoding the variants of the gene of interest, the variants collectively comprising one or more genetic mutations in the gene of interest, wherein each nucleic acid molecule is uniquely bar coded with additional genetic material and a plurality of separately barcoded nucleic acid molecules encode a same variant;
introducing the library into a population of eukaryotic cells such that the eukaryotic cells express the variants of the gene of interest and are exposed to a probe;
assaying the molecular function or cell process by measuring one or more of a reporter output or cellular phenotype in the population of cells, wherein the reporter output or cellular phenotype is associated with activity of the probe;
sorting the population of cells into a plurality of pools based on a change in a measurable activity of the probe or the reporter output;
obtaining sequence information from the plurality of pools to identify the unique barcodes;
grouping the barcodes by associating each barcode or barcode group with a corresponding variant in the gene of interest; and
for each variant, determining a distribution of reads or an activity measurement across the plurality of pools by aggregating read counts from the multiple independently barcoded nucleic acid molecules encoding the same variant.

2. The method of Claim 1, further comprising determining the effect of the variant on the molecular function or cell process.

3. The method of Claim 1, wherein determining the distribution of reads or the activity measurement across the plurality of pools for each variant comprises using concordance or discordance among the multiple independently barcoded nucleic acid molecules encoding the same variant to identify, reduce, exclude, correct for, or rule out false measurements or assay artifacts.

4. The method of claim 1, further comprising, for each variant, using comparisons of activity measurements obtained from the multiple independently barcoded nucleic acid molecules encoding the same variant to identify one or more barcode measurements that are inconsistent with other barcode measurements associated with the same variant, and excluding the identified barcode measurements as false measurements when determining the distribution of reads or activity measurement for the variant.

5. The method of any one of claims 1-4, wherein the eukaryotic cells are mammalian cells.

6. The method of Claim 5, wherein the mammalian cells comprise an immortalized cell line selected from LentiX293T cells and Jurkat cells.

7. The method of any one of claims 1-6, wherein a measurable activity of the reporter output comprises measuring expression of a fluorescent protein encoded by a reporter gene operably linked to a promoter that is activated by the molecular function or cell process.

8. The method of Claim 7, wherein the fluorescent protein is green fluorescent protein (GFP) and the promoter is a human immunodeficiency virus (HIV) long terminal repeat (LTR) promoter.

9. The method of Claim 1, wherein obtaining sequence information from the plurality of pools comprises sequencing amplicons that include the unique barcodes.

10. The method of any one of claims 1-9, wherein each unique barcode comprises 32 contiguous nucleotides and each variant has a total sequencing coverage between about 2,000X and about 90,000X across the plurality of pools.

11. The method of any one of claims 1-10, wherein determining the activity measurement for each variant comprises calculating, for each barcode associated with the variant, a ratio of reads in a positive reporter output to the total number of reads across positive and negative reporter outputs, and averaging the ratios across the multiple independently barcoded nucleic acid molecules encoding the same variant.

12. The method of any one of claims 1-11, wherein sorting the population of cells into the plurality of pools comprises flow cytometry based sorting into at least two pools corresponding to negative reporter output and positive reporter output.

13. The method of any one of claims 1-12, wherein determining, for each variant, the distribution of reads or the activity measurement across the plurality of pools comprises applying a minimum read depth threshold per barcode, such that barcodes with a read depth below a predetermined reads per million threshold are excluded from the analysis.

14. The method of Claim 13, wherein the predetermined reads per million value is 2.5 reads per million.
